(19) Europäisches Patentamt
European Patent Office
Office européen des brevets

(11) **EP 3 706 584 B1**

(12) **EUROPÄISCHE PATENTSCHRIFT**

(45) Veröffentlichungstag und Bekanntmachung des Hinweises auf die Patenterteilung:
**24.04.2024   Patentblatt 2024/17**

(21) Anmeldenummer: **17808345.7**

(22) Anmeldetag: **09.11.2017**

(51) Internationale Patentklassifikation (IPC):
**A23L 27/00** (2016.01)    **A23L 27/29** (2016.01)
**B01D 61/00** (2006.01)    **B01D 61/02** (2006.01)
**B01D 69/00** (2006.01)    **B01D 69/14** (2006.01)
**A23F 3/16** (2006.01)    **A23F 5/24** (2006.01)
**A23L 19/00** (2016.01)    **A23L 27/10** (2016.01)
**B01D 71/56** (2006.01)

(52) Gemeinsame Patentklassifikation (CPC):
**A23L 27/29; A23F 3/16; A23F 5/24; A23L 19/00;
A23L 27/00; A23L 27/10; A23L 27/11;
B01D 61/002; B01D 69/144;** B01D 71/56

(86) Internationale Anmeldenummer:
**PCT/EP2017/078735**

(87) Internationale Veröffentlichungsnummer:
**WO 2019/091556 (16.05.2019 Gazette 2019/20)**

(54) **HERSTELLUNG VON AROMAKONZENTRATEN DURCH OSMOSE**

PREPARATION OF AROMA CONCENTRATES VIA OSMOSIS

PROCÉDÉ POUR LA PRÉPARATION DE CONCENTRÉS AROMATIQUES PAR OSMOSE

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**

(43) Veröffentlichungstag der Anmeldung:
**16.09.2020   Patentblatt 2020/38**

(73) Patentinhaber: **Symrise AG
37603 Holzminden (DE)**

(72) Erfinder:
• KIEFL, Johannes
  91413 Neustadt an der Aisch (DE)
• UTERMÖHLE, Claudia
  37170 Uslar (DE)
• BRENNECKE, Stefan
  37620 Halle (DE)

(74) Vertreter: **Eisenführ Speiser
Patentanwälte Rechtsanwälte PartGmbB
Gollierstraße 4
80339 München (DE)**

(56) Entgegenhaltungen:
EP-A1- 2 075 320    WO-A1-2014/108827
DK-A1- 201 770 753    US-A- 4 952 751
US-A1- 2012 152 841    US-A1- 2016 016 127
US-A1- 2017 259 217

• Watanabe Michiko ET AL: "Freeze Concentration of Some Foodstuffs Using Ice Nucleation-active Bacterial Cells Entrapped in Calcium Alginate Gel", Agricultural and Biological Chemistry, vol. 53, no. 10, 1 October 1989 (1989-10-01), pages 2731-2735, XP55859668, JP ISSN: 0002-1369, DOI: 10.1080/00021369.1989.10869739

**Beschreibung**

**Gebiet der Erfindung**

Die vorliegende Erfindung betrifft ein Verfahren zur Herstellung eines Aromastoff-Konzentrates, ein Aromastoff-Konzentrat, herstellbar nach dem erfindungsgemäßen Verfahren, das frei ist von störenden Aromakomponenten mit einem OAV (odor activity value) $\geq$ 1 und das keine Lösungsmittelzusätze enthält sowie die Verwendung der Aromastoff-Konzentrate.

**Stand der Technik**

Aromastoffe spielen bei zahlreichen industriellen Verfahren eine große Rolle. Dabei sind dies einmal Verfahren, die allein auf die Gewinnung von Aromastoffen in Form von Essenzen aus Früchten, Gemüse, Gewürzen, Blüten und ähnlichen gerichtet sind und zum anderen Verfahren zur Herstellung oder Veredelung von Nahrungs- und Genussmitteln, bei denen die Erhaltung der natürlichen Aromastoffe, wie Duft- und Geschmacksstoffe, die Qualität der Produkte bestimmt bzw. diese Produkte erst genussfähig macht.

Das aus einer bestimmten Quelle, z. B. einer Frucht stammende Aroma ist kein chemisch einheitlicher Stoff, sondern setzt sich zusammen aus einer Vielzahl von unterschiedlichen chemischen Komponenten, die erst in ihrer Gesamtheit das sensorische Ergebnis des natürlichen Aromas eines Lebensmittels ergeben. Bei der Handhabung von aromastoffhaltigen Medien bzw. bei der Verarbeitung von Lebensmitteln besteht erfahrungsgemäß die Gefahr, dass Teile der natürlichen Mischung verlorengehen oder abgebaut werden und damit der natürliche Gehalt gemindert oder gar zerstört wird, was zu einem sogenannten "Off-Flavour" führt. Dies ist insbesondere der Fall, wenn aromastoffhaltige Lösungen konzentriert werden.

Bei der Gemüse- und Obstsaftherstellung sowie bei der Extraktion von Lebensmittel entstehen industriell bedeutsame Mengen geruchs- und geschmacksintensiver wässriger Lösungen, die aufkonzentriert und als Konzentrat zugegeben das selbige Lebensmittel re-aromatisieren oder andere Lebensmittel aromatisieren. Beispielsweise wird bei der Gewinnung von Obst- oder Gemüsesaftkonzentrat ein aromatisches Brüdenkondensat gewonnen. Dabei werden durch Verdampfung frisch gepresste Obst- und Fruchtsäfte aus Stein-, Kern- und Beerenobst sowie auch Säfte von Citrus- und tropischen Früchten aufkonzentriert und damit haltbar gemacht. Während des Verdampfungsvorganges werden dem Dünnsaft leicht flüchtige Aromastoffe in Form eines Brüdenkondensates entzogen, welches danach aufkonzentriert wird. Vor der Abfüllung wird das Obst- oder Gemüsesaftkonzentrat rückverdünnt und das zuvor abgetrennte Aromakonzentrat zur Rekonstitution des Aromas dem Obst- oder Gemüsesaft zugegeben. Obst- oder Gemüsesaftkonzentrat, das nur ein Sechstel bis ein Achtel seines ursprünglichen Volumens aufweist, spart Lager- und Transportkosten. Eine weitere wichtige Anwendung für die Eindampftechnik in der Saftindustrie ist die Aufkonzentrierung von Extrakten verschiedenster Ausgangsstoffe. So werden beispielsweise aus dem Fruchtfleisch und den Schalen von Citrusfrüchten die Saftreste und Öle extrahiert, durch Eindampfen aufkonzentriert, danach separiert und so weiterverarbeitet. Auch in anderen Bereichen der Getränkeindustrie werden Eindampfanlagen eingesetzt, zum Beispiel in der Brauindustrie zum Konzentrieren von Malzextrakt, Bierhefe, HefeExtrakt, Hopfenextrakt.

Rückverdünnte Obst- oder Gemüsesäfte müssen sich an dem Geschmack von frischgepressten Säften messen. Durch das gestiegene Verbraucherinteresse nach authentischem Geschmackserlebnis und gesundheitsbewusster Ernährung sind bestehende Verfahren zur Aufkonzentrierung wässriger Lösungen nur noch eingeschränkt nutzbar.

Seit Jahrzehnten werden mittels destillativer Verfahren wässrige Lösungen aus Lebensmitteln mit geruchs- und geschmacksaktiven Substanzen aufkonzentriert. Trotz Anwendung schonender Prozessparameter wie beispielsweise die Verwendung von Vakuum zur Verringerung der Dampfdrücke der flüchtigen Komponenten können bereits bei Temperaturen von 30 °C störende Geschmackskomponenten gebildet und erwünschte Geschmackskomponenten abgebaut werden. So können beispielsweise durch Destillation durch die thermische Belastung des wässrigen Produkts Kochnoten entstehen, die nicht als authentisches Geschmackserlebnis angesehen werden. So bilden sich bei der Aufkonzentrierung von Erdbeer-Brüden-kondensaten Kochnoten, die den Verbraucher an Konfitüre und nicht an den Verzehr frischer Erdbeeren erinnern.

Ein neueres Verfahren betrifft die Gewinnung eines Aromakonzentrates durch Adsorption von Geschmacksstoffen an Festphasenmaterial, wie dies beispielsweise in der EP 2 075 320 A1 beschrieben wird. Bei der adsorptiven Aufkonzentrierung von wässrigen Lösungen, wie sie beispielsweise in der EP 0 082 284 A1 beschrieben wird, wird die wässrige Lösung durch eine Schüttung eines mit organischen Resten oberflächenmodifizierten porösen Sorbens geschickt und die Schüttung danach mit einer im Vergleich zur wässrigen Lösung geringen Menge eines organischen Lösungsmittels eluiert. Allerdings müssen bei diesem Verfahren Lösungsmittel zur Desorption eingesetzt werden, wie beispielsweise Methanol, das als toxikologisch bedenklich gilt. Ein weiterer Nachteil dieser Verfahren des Standes der Technik ist, dass sich durch die Selektivität des Adsorbens sowie des Lösungsmittels zur Elution/Desorption das Aromaprofil verschieben kann.

Die lösemittelfreie sowie nicht-thermische Aufkonzentrierung geruchs- und geschmacksintensiver wässriger Lösungen beschäftigt die Lebensmittelindustrie deshalb seit Längerem. Insbesondere wird an membrangestützten Verfahren gearbeitet, um Wasser abzutrennen und wertgebende Komponenten anzureichern ohne dabei Lösemittel zu verwenden. Beispielsweise konnte mit einer Kombination von Ultrafiltration und Umkehrosmose Wasser aus wässrigen Lösungen der Citrusverarbeitung entfernt werden (Braddock, R.J. "Ultrafiltration and Reverse Osmosis Recovery of Limonene from Citrus Processing Waste Streams", Journal of Food Science, 1982, 47(3), 946 - 948). Dabei wurden Hohlfaser-Flachmembranen, die aus klassischen Werkstoffen wie Celluloseacetat, Polysulfon und Polytetrafluorethylen bestehen, verwendet. Bei der Umkehrosmose werden jedoch üblicherweise Drücke von 10 bis über 100 bar verwendet, die allgemein bekannt zur Beschleunigung von Abbaureaktionen führen, was das sensorische Profil der erhaltenen Konzentrate negativ beeinflusst.

In der WO 2017 080852 A1 wird ein Membranverfahren zur Aufkonzentrierung von wässrigen Kaffeextrakten beschrieben, die sensorisch stärker an frisch gebrühtem Kaffee erinnern als herkömmlich über Evaporation, Sprühtrocknung oder Gefriertrocknung gewonnene Instant Kaffees. Dabei dringen neben Wasser weitere Stoffe mit geringer Molmasse d.h. kleiner 1 kDa wie kleine sensorisch aktive Stoffe durch die Membran und gehen dadurch verloren. Ein hoher Verlust an Aromastoffen, die in der Regel ein Molekulargewicht von 100 - 300 Da aufweisen, wird akzeptiert um einen hohen Permeationsfluss von 8,3 l/m$^2$/h bei 10 - 20 bar und damit schnelle Entwässerung zu erreichen. Die Beispiele zeigen, dass Membrane aus herkömmlichen Materialien wie Celluloseacetat, Polysulfon, Polyamid, Polytetrafluorethylen, etc. ungeeignet sind, wässrige Lösungen in einem stabilen und kostengünstigen Prozess ohne Veränderung des Aromaprofils aufzukonzentrieren. Entweder ist durch die geringe Permeabilität der Flux zu gering, um den Prozess wirtschaftlich zu betreiben bzw. der Transmembrandruck so hoch, dass sich das Aroma abbaut oder sich durch die gute Permeabilität eine schlechte Selektivität ergibt, wodurch die Aromastoffe mit dem Wasser verloren gehen.

Seit kurzem sind biomimetische Membrane auf dem Markt, die mit Aquaporinen durchsetzt sind. Aquaporine sind zellmembranständige und in lebenden Organismen weit verbreitete Proteine, die unter bestimmten Bedingungen einen Wasserkanal bilden, um Wassermoleküle durchzulassen und gelöste anorganische sowie organische Stoffe zurückzuhalten.

Wie durch Tang et al. "Desalination by biomimetic aquaporin membranes: Review of status and prospects", Desalination 308, 2013, beschrieben, zeigen in der Natur Aquaporine eine selektive Permeabilität beispielsweise gegenüber Wasser, Glycerol oder Salzen wobei die Funktionsweise der Selektivität in vielerlei Dingen noch unklar ist. Erschwerend kommt hinzu, dass durch die industrielle Verarbeitung zu Membranen, wie die rekombinante Produktion von AQPs und das Einbetten in dem Membranpolymer, sowie durch die Prozessinstabilität, die Leistung der Membran abnimmt. Darin findet sich keine Lehre über die Filtrierbarkeit von wässrigen Lösungen mit organischen Geschmacks- und Geruchsstoffen.

In der WO 2014 108827 A1 ist beispielsweise beschrieben, dass Aquaporin-Membrane für die Dialyse von Blut eingesetzt werden können, im Speziellen zur Abtrennung von Humanmetaboliten wie Harnstoff, p-Kresylsulfat und Peptiden bis zu einem Molekulargewicht von 692 Da. Allerdings findet sich keine Lehre über die gezielte selektive An- oder Abreicherung von organischen Geschmacks- und Geruchsstoffen zur Herstellung einer geschmacks- oder aromagebenden Zubereitung.

Die US 2016 016127 A1 offenbart die Verwendung von Aquaporin-Membranen zur Aufkonzentrierung von flüssigen Lebensmitteln, um auf schonende Weise Wasser aus Lösungen von z.B. kleinen organischen Verbindungen zu entfernen. Die eingesetzten Membranen sind Dünnfilm-Komposit-Membranen.

Die US 2017 259217 A1 beschreibt die Aquaporin-Membranen und deren möglich Anwendung in der Biotechnologie sowie dem Lebensmittel- und Getränkebereich, z.B. zur Abtrennung von Bakterien, Konzentration von Molke, Frucht- oder Gemüsesaft, etc. Die Aquaporine werden durch β-Proteine stabilisiert.

Die US 2012 152841 A1 offenbart eine Methode zur Extraktion von Wasser aus wässrigen Lösungen wie z.B. Wein, Frucht- oder Gemüsesäften, Milch, etc. mittels Aquaporin-enthaltenden Membranen und Forward-Osmose. Es wird eine flüssige Membran-Matrix verwendet.

Die US 4 952 751 offenbart ein Verfahren zum Aufkonzentrieren von Fruchtsaftdestillaten mittels Pervaporationsmembranen.

DK 2017 70753 A1 beschreibt die Aufkonzentrierung von Kaffeeextrakt mittels Aquaporin-Membranen.

Ziel der vorliegenden Erfindung war es, ein Verfahren zu entwickeln, mit dem aus einer geruchs- und geschmacksaktiven wässrigen Lösung aus einem Lebensmittel ein Aromastoff-Konzentrat ohne Verluste an diesen wertgebenden Geschmacks- und Geruchsstoffen hergestellt und das sensorische Profil der Ausgangsprobe beibehalten werden kann.

## Beschreibung der Erfindung

Die vorliegende Problemstellung wird durch die Gegenstände der unabhängigen Patentansprüche gelöst. Bevorzugte Ausgestaltungen ergeben sich aus dem Wortlaut der abhängigen Patentansprüche sowie der nachfolgenden Beschreibung.

Ein erster Gegenstand der vorliegenden Erfindung betrifft ein Verfahren zur Herstellung eines Aromastoff-Konzentrates, umfassend die folgenden Schritte:

- Bereitstellen einer Aromastoff umfassenden wässrigen Ausgangslösung aus einem Lebensmittel, wobei die wässrige Ausgangslösung ein Brüdenkondensat aus Obst oder Gemüse ist,

- Aufkonzentration der wässrigen Ausgangslösung durch Osmose mit einer semipermeablen biomimetischen Membran, und

- Bildung eines Aromastoff-Konzentrats als Retentat,

worin die semipermeable biomimetische Membran umfasst:

Vesikel aus Liposomen oder Polymersomen, in die jeweils mindestens ein Aquaporin-Protein eingebaut ist;
eine Dünnfilm-Verbundmatrix, in der die Vesikel eingebettet sind; und
eine Trägerschicht;
wobei das Verfahren bei einer Temperatur im Bereich von 10 bis 40 °C durchgeführt wird; und
worin die wässrige Ausgangslösung um den Faktor 20 bis 1.000 aufkonzentriert wird.

Ein weiterer Gegenstand der vorliegenden Erfindung betrifft ein Aromastoff-Konzentrat, erhältlich nach einem erfindungsgemäßen Verfahren, das kein Off-flavour aufweist und frei ist von störenden Aromakomponenten, insbesondere frei ist von störenden Aromakomponenten mit einem OAV (odor activity value) $\geq$ 1.

Des Weiteren betrifft einen Gegenstand der vorliegenden Erfindung ein Aromastoff-Konzentrat wie oben beschrieben, das keine Lösungsmittelzusätze enthält.

Ein weiterer Aspekt der vorliegenden Erfindung betrifft die Verwendung des erfindungsgemäßen Aromastoff-Konzentrats zur Aromatisierung oder zur Rekonstitution des Aromas von Lebensmitteln, Getränkeprodukten, Halbfertigprodukten, Mundhygiene-produkten, kosmetischen oder pharmazeutischen Produkten.

**Beschreibung der Figuren**

Figur 1 ist eine schematische Darstellung des Aufbaus einer erfindungsgemäß verwendeten Aquaporin-Membran

Figur 2 ist eine schematische Darstellung des Prinzips der Forward-Osmose

Figur 3 ist eine schematische Darstellung des Verfahrens am Beispiel eines Kaffee-Extraktes (nicht Teil der vorliegenden Erfindung).

Figuren 4 a und 4 b sind ein Chromatogrammvergleich von einem Teeextrakt (Wasserphase) und dem rückverdünnten Konzentrat des gleichen Teeextraktes (Aufkonzentrieren von Teeextrakt ist nicht Teil der vorliegenden Erfindung).

Figur 5 ist die Darstellung einer sensorischen Profilierung von Wasserphase und rückverdünntem Konzentrat aus der Aquaporin-Membranfiltration von gelbem Pfirsich (Aufkonzentrieren von wässrigen Pfirsichextrakten/säften ohne ein Brüdenkondensat im Verfahren zu erhalten ist nicht Teil der vorliegenden Erfindung).

Figur 6 ist die Darstellung einer sensorischen Profilierung von Wasserphase und rückverdünntem Konzentrat aus der Aquaporin-Membranfiltration von Erdbeere

**Detaillierte Beschreibung der Erfindung**

Bei dem erfindungsgemäßen Verfahren zur Herstellung eines Aromastoff-Konzentrates wird in einem ersten Schritt eine wässrige Ausgangslösung aus einem Lebensmittel bereitgestellt, wobei die wässrige Ausgangslösung ein Brüdenkondensat aus Obst oder Gemüse ist.

Lebensmittel sind alle Stoffe oder Erzeugnisse, die dazu bestimmt sind, dass sie in verarbeitetem, teilweise verarbeitetem oder unverarbeitetem Zustand von Menschen aufgenommen werden. Zu Lebensmitteln zählen auch Getränke, sowie alle Stoffe, einschließlich Wasser, die dem Lebensmittel bei seiner Herstellung oder Be- oder Verarbeitung absichtlich zugesetzt werden. Den Lebensmitteln zugeordnet werden auch Genussmittel. Als Genussmittel im engeren Sinne werden Lebensmittel bezeichnet, die nicht in erster Linie wegen ihres Nährwertes und zur Sättigung konsumiert werden, sondern wegen ihrer anregenden Wirkung oder ihres Geschmacks. Zu den Genussmitteln zählen beispielsweise Kaffee, Tee, Kakao und Gewürze. In der Beschreibung werden pflanzliche oder pilzliche Rohstoffe, die nicht für den direkten Verzehr aber zur Herstellung von Aromazubereitungen geeignet sind und üblicherweise gebraucht werden, auch zu den Lebensmitteln gezählt.

Unter wässriger Ausgangslösung eines Lebensmittels wird entweder ein Lebensmittel verstanden, das von Natur aus in wässriger Form vorliegt, wie beispielsweise Milch, eine wässrige Lösung aus einem Lebensmittel, die durch Zusatz von Wasser zu einem Lebensmittel erhalten wird, ein wässriger Extrakt aus einem Lebensmittel, der durch Zusatz von Wasser zu einem Lebensmittel, wie beispielsweise verzehrfähigen Pflanzen, wie beispielsweise Tee oder Kaffee, erhalten wird, oder ein Direktsaft aus Obst oder Gemüse. Eine wässrige Ausgangslösung eines Lebensmittels schließt auch typische wässrige Lösungen ein, die bei der Be- oder Verarbeitung eines Lebensmittels anfallen, beispielsweise ein aromatisches Brüdenkondensat, welches bei der Herstellung von Saftkonzentraten aus Obst oder Gemüse mittels Saftverdampfung anfällt.

Zur Herstellung eines Direktsaftes wird zunächst reifes Obst oder Gemüse gewaschen, ausgelesen und in einem Mühlwerk oder einer Fräse zur sogenannten Maische zerkleinert. Die Maische wird gegebenenfalls vor der Weiterverarbeitung mit speziellen Enzymen wie beispielsweise Pektinasen, Cellulasen und Hemicellulasen entpektinisiert, um eine bessere Saftausbeute zu erhalten. Die Entsaftung erfolgt anschließend in einer Presse bei einem Druck von 5 bis 20 bar oder durch Zentrifugation. Der so erhaltene Rohsaft wird vor der Weiterverarbeitung zu Konzentrat depektinisiert, geklärt und filtriert.

Alternativ dazu kann der Rohsaft zu Konzentrat weiterverarbeitet werden. Vor der Weiterverarbeitung zu Konzentrat muss der Saft depektinisiert, geklärt und filtriert werden. Neben der Gefrierkonzentrierung wird zur Herstellung von Saftkonzentrat das Verfahren der Verdampfung eingesetzt. Bei der Konzentrierung durch Verdampfen entsteht ein Brüdenkondensat, welches die Aroma- und Geschmacksstoffe des Ausgangsproduktes enthält, und als wässrige Ausgangslösung in dem erfindungsgemäßen Verfahren zum Einsatz kommt.

Es kommt in dem erfindungsgemäßen Verfahren ein Brüdenkondensat aus Obst oder Gemüse als wässrige Ausgangslösung zum Einsatz.

In dem zweiten Schritt des erfindungsgemäßen Verfahrens wird die oben beschriebene wässrige Ausgangslösung aus einem Lebensmittel durch Osmose mit einer semipermeablen biomimetischen Membran aufkonzentriert.

Als Osmose wird in den Naturwissenschaften der gerichtete Fluss von molekularen Teilchen durch eine selektive oder semipermeable Trennschicht bzw. Membran bezeichnet. Insbesondere wird Osmose beschrieben als die spontane Passage von Wasser oder eines anderen Lösungsmittels durch eine semipermeable Membran, die für das Lösungsmittel, jedoch nicht die darin gelösten Stoffe durchlässig ist.

Die Osmose in dem erfindungsgemäßen Verfahren kann als Forward-Osmose oder als Reverse-Osmose bzw. Umkehrosmose bei niedrigem Druck durchgeführt werden. Bevorzugt wird in dem erfindungsgemäßen Verfahren die Forward-Osmose.

Die Reverse-Osmose bzw. Umkehrosmose ist ein physikalisches Verfahren zur Konzentrierung von in Flüssigkeiten gelösten Stoffen, bei der mit Druck der natürliche Osmose-Prozess umgekehrt wird. Das Wirkungsprinzip besteht darin, dass das Medium, in dem die Konzentration eines bestimmten Stoffes verringert werden soll, durch eine halbdurchlässige (semipermeable) Membran von dem Medium getrennt, in dem die Konzentration erhöht werden soll. Dieses wird einem Druck ausgesetzt, der höher sein muss als der Druck, der durch das osmotische Verlangen zum Konzentrationsausgleich entsteht. Dadurch können die Moleküle des Lösungsmittels gegen ihre "natürliche" osmotische Ausbreitungsrichtung wandern. Das Verfahren drückt sie in das Kompartiment, in dem gelöste Stoffe weniger konzentriert vorliegen.

Trinkwasser hat einen osmotischen Druck von weniger als 2 bar, der angewendete Druck für die Umkehrosmose von Trinkwasser beträgt 3 bis 30 bar, je nach verwendeter Membran und Anlagenkonfiguration. Die osmotische Membran, die nur die Trägerflüssigkeit (Solvent) durchlässt und die gelösten Stoffe (Solute) zurückhält, muss diesen hohen Drücken standhalten können. Wenn der Druckunterschied das osmotische Gefälle mehr als ausgleicht, passen die Solventmoleküle wie bei einem Filter durch die Membran, während die "Verunreinigungsmoleküle" zurückgehalten werden. Im Gegensatz zu einem klassischen Membranfilter verfügen Osmose-Membrane nicht über durchgehende Poren. Vielmehr wandern die Ionen und Moleküle durch die Membran hindurch, indem sie durch das Membranmaterial diffundieren.

Der osmotische Druck steigt mit zunehmendem Konzentrationsunterschied. Wird der osmotische Druck gleich dem angelegten Druck, kommt der Prozess zum Stehen. Es liegt dann ein osmotisches Gleichgewicht vor. Ein stetiger Abfluss des Konzentrats kann das verhindern.

Die Forward-Osmose ist ein osmotisches Verfahren, das eine semipermeable Membran verwendet, um die Trennung von Wasser aus einer Lösung mit gelösten Stoffen zu bewirken. Die treibende Kraft für diese Trennung ist ein osmotischer Druckgradient zwischen einer Lösung hoher Konzentration (Zuglösung), die auch als "Draw Solution" bezeichnet wird, und eine Lösung geringerer Konzentration, die auch als "Feed Solution" bezeichnet wird. Ein osmotischer Druckgradient wird verwendet, um einen Fluss von Wasser durch die Membran in die Lösung hoher Konzentration zu induzieren, wodurch die Lösung geringer Konzentration effektiv konzentriert wird.

Sowohl bei der Reverse-Osmose als auch bei der Forward-Osmose wird mittels einer biomimetischen Membran, die nachfolgend näher beschrieben wird, in dem erfindungsgemäßen Verfahren dem Brüdenkondensat aus Obst oder Gemüse Wasser entzogen, so dass eine Aufkonzentration der wässrigen Ausgangslösung erfolgt.

Vorzugsweise wird in dem erfindungsgemäßen Verfahren die Aufkonzentration des Brüdenkondensats aus Obst oder Gemüse mit einer biomimetischen Membran als Forward-Osmose durchgeführt.

Noch mehr bevorzugt wird die Aufkonzentration des Brüdenkondensats aus Obst oder Gemüse mit einer biomimetischen Membran als Forward-Osmose gegen eine Osmose-Lösung durchgeführt. Dazu wird die osmotisch aktive Lösung (Draw Solution) im Gegenstrom mit dem Brüdenkondensat aus Obst oder Gemüse gefahren, wodurch diesem das Wasser entzogen wird.

Üblicherweise kommen bei der Forward-Osmose in dem erfindungsgemäßen Verfahren Osmose-Lösungen zum Einsatz, die einen oder mehrere chemische(n) Bestandteil(e) aus der folgenden Gruppe umfasst: Salze, bestehend aus einem Kation, das ausgewählt ist aus der Gruppe, die besteht aus Ammonium, Natrium, Kalium, Calcium und Magnesium; und aus einem Anion, das ausgewählt ist aus der Gruppe, die besteht aus Acetat, Chlorid, Citrat, Hydrogencarbonat, Formiat, Lactat, Malat, Propionat, Sulfat, Succinat und Tartrat. Daneben kommen Salze von Aminosäuren und Einfachzucker wie Glucose, Fructose und Saccharose zum Einsatz.

Bevorzugt werden als osmotisch aktive Lösungen, solche Lösungen eingesetzt, die NaCl, $MgCl_2$, KCl, K-Lactat, $NH_4HCO_3$ oder Saccharose umfassen. In dem erfindungsgemäßen Verfahren werden am meisten bevorzugt Osmose-Lösungen, die NaCl, K-Lactat oder Saccharose umfassen. Besonders bevorzugt ist eine Osmose-Lösung, die NaCl umfasst.

Die Osmose-Lösung, die in dem erfindungsgemäßen Verfahren zum Einsatz kommt, hat eine Ausgangs-Konzentration der chemischen Bestandteile im Bereich von 0,05 bis 4 M, vorzugsweise im Bereich von 0,5 bis 3 M.

Das erfindungsgemäße Verfahren zeichnet sich dadurch aus, dass in dem Schritt zur Aufkonzentrierung des Brüdenkondensats aus Obst oder Gemüse mittels eines des zuvor beschriebenen Osmose-Verfahrens eine semipermeable biomimetische Membran gemäß den Ansprüchen verwendet wird. Bei diesen biomimetischen Membranen handelt es sich vorzugsweise um solche Membrane, wie sie von Joachim Habel et al. in "Membranes "Aquaporin-based biomimetic polymeric mambrances: approaches and challenges"; 2015, 5, 307 - 351; ISSM 2077-0375", insbesondere den Seiten 312 - 319, beschrieben werden. Die Offenbarung der dort beschriebenen biomimetischen Polymer-Membrane auf Aquaporin-Basis wird durch die spezifische Inbezugnahme auf die darin offenbarten Membranen in vollem Umfang in die vorliegende Anmeldung übernommen.

Derartige biomimetische Membrane, die mit Aquaporinen durchsetzt sind, sind bereits im Handel erhältlich. Sie werden von der Firma Aquaporin A/S, Dänemark, hergestellt und unter dem Handelsnamen Aquaporin Inside R vertrieben. Solche Aquaporin-Membranen werden beispielsweise bei der Dialyse von Blut oder Entsalzung von Meerwasser eingesetzt.

Die in dem erfindungsgemäßen Verfahren verwendeten semipermeablen biomimetischen Aquaporin-Membrane sind dadurch gekennzeichnet, dass sie

- Vesikel aus Liposomen oder Polymersomen, in die jeweils mindestens ein Aquaporin-Protein eingebaut ist;
- eine Dünnfilm-Verbundmatrix, in der die Vesikel eingebettet sind; und
- eine Trägerschicht

umfassen.

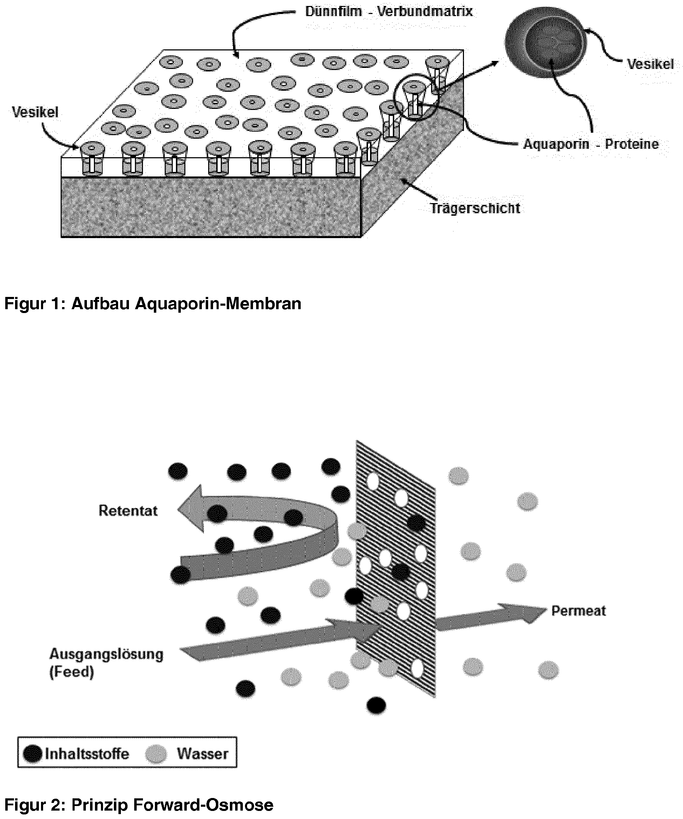

Der Aufbau der oben beschriebenen semipermeablen biomimetischen Aquaporin-Membran ist in Figur 1 gezeigt.

Wie aus Figur 1 ersichtlich ist, umfasst die Aquaporin-Membran in der aktiven Schicht Vesikel, in die Aquaporine eingebaut sind. Aquaporine sind zellmembranständige und in lebenden Organismen weit verbreitete Proteine, die unter bestimmten Bedingungen einen Wasserkanal bilden, um Wassermoleküle durchzulassen und gelöste anorganische sowie organische Stoffe in der Zelle zurückzuhalten. Sie werden daher auch Wasserkanäle genannt. In der Aquaporin-Membran bilden die Aquaporine Wasserkanäle, die bevorzugt Wassermoleküle passieren lassen, während die Passage anderer Moleküle, ungeachtet ihrer Größe, ihres Molekulargewichts und ihrer chemischen Struktur, idealerweise blockiert wird.

Die Vesikelmatrix besteht aus mindestens einem Liposom oder mindestens einem Polymersom. Das Liposom umfasst: Lipide wie DPhPC, DOPC, gemischte Sojabohnen-Lipide, Asolectin oder gemischte Lipide von E. coli. Das Polymersom umfasst: Triblockcopolymere des hydrophilen-hydrophoben-hydrophilen Typs A-B-A, A-B-C, C-B-A, worin A steht für PMOXA, B steht für PDMS und C steht für PEO, Diblockcopolymere des hydrophilen-hydrophoben Typs A-B, worin A steht für PB und B steht für PEO, oder Kombinationen daraus.

Die Aquaporine umfassen bevorzugt nach der Erfindung mindestens ein Protein, das ausgewählt ist aus der Gruppe, die besteht aus AQPO, AqpZ, SoPIP2, AQP10 und deren Isoformen. Besonders bevorzugt ist AQPO.

Wie weiter aus der Figur 1 ersichtlich ist, sind die Vesikel, die die Aquaporine enthalten, an der Oberseite einer Dünnfilm-Verbundmatrix, eingebaut. Die Dünnfilm-Verbundmatrix wird hergestellt durch Polymerisation einer wässrigen Lösung eines Amins mit einer Lösung eines Säurechlorids in einem organischen Lösungsmittel. Die Aquaporin-Wasserkanal-Vesikel werden in diese wässrige Lösung eingearbeitet.

Die Dünnfilm-Verbundmatrix wiederum ist aufgetragen auf eine poröse Trägerschicht. Die Trägerschicht ist bevorzugterweise ein Polyethersulfon.

Die Herstellung dieser semipermeablen biomimetischen Membranen, weitere Zusammensetzungen und physikalische und chemische Eigenschaften sind beispielsweise beschrieben in der WO 2014108827 A1, insbesondere den Seiten 6 bis 33.

In der nachfolgenden Tabelle 1 sind beispielhaft Zusammensetzungen solcher Aquaporin-Membrane aufgeführt:

**Tabelle 1:** Zusammensetzung von Aquaporin-Membranen

| Polymer | Membran-Protein | Durchlässigkeit |
|---|---|---|
| $PMOXA_{20}$-$PDMS_{41}$-$PMOXA_{20}$ | AQP0 | $H_2O$ |
| $PMOXA_{12}$-$PDMS_{54}$-$PMOXA_{12}$ | AqpZ | $H_2O$ |
| $PMOXA_8$-$PDMS_{55}$-$PMOXA_8$ | AqpZ | $H_2O$ |
| $PMOXA_{12}$-$PDMS_{55}$-$PMOXA_{12}$ | AQP0 | $H_2O$ |
| $PMOXA_{12}$-$PDMS_{55}$-$PMOXA_{12}$ | AqpZ | $H_2O$ |
| $PMOXA_8$-$PDMS_{60}$-$PMOXA_8$ | AqpZ | $H_2O$ |
| $PMOXA_{15}$-$PDMS_{68}$-$PMOXA_{15}$ | AqpZ | $H_2O$ |
| $PMOXA_{20}$-$PDMS_{75}$-$PMOXA_{20}$ | AqpZ | $H_2O$ |
| $PMOXA_{110}$-$PDMS_{40}$-$PEO_{25}$ | AQP0 | $H_2O$ |
| $PMOXA_{45}$-$PDMS_{40}$-$PMOXA_{67}$ | AQP0 | $H_2O$ |
| $PB_{12}$-$PEO_{10}$ | AQP0 | $H_2O$ |
| $PB_{12}$-$PEO_{10}$ | AqpZ | $H_2O$ |
| $PB_{12}$-$PEO_{10}$ | SoPIP2;1 | $H_2O$ |
| $PB_{22}$-$PEO_{14}$ | AQP0 | $H_2O$ |
| $PB_{22}$-$PEO_{23}$ | AqpZ | $H_2O$ |
| $PB_{22}$-$PEO_{23}$ | SoPIP2; 1 | $H_2O$ |
| $PB_{29}$-$PEO_{16}$ | AQP10 | $H_2O$ |
| $PB_{35}$-$PEO_{14}$ | AqpZ | $H_2O$ |
| $PB_{35}$-$PEO_{14}$ | SoPIP2; 1 | $H_2O$ |
| $PB_{43}$-$PEO_{32}$ | AQP10 | $H_2O$ |
| $PB_{46}$-$PEO_{30}$ | AqpZ | $H_2O$ |
| $PB_{46}$-$PEO_{32}$ | AQP10 | $H_2O$ |
| $PB_{92}$-$PEO_{78}$ | AQP10 | $H_2O$ |

Die Funktionsweise der Aufkonzentrierung eines Brüdenkondensats aus Obst oder Gemüse mit einer semipermeablen biomimetischen Aquaporin-Membran durch Forward-Osmose gemäß der Erfindung wird nun anhand von Figur 2 näher beschrieben. Bei der Forward-Osmose ist die wässrige Ausgangslösung, die beispielsweise wertgebende Aroma- und Geschmacksstoffe enthält, räumlich durch die semipermeable biomimetische Aquaporin-Membran von der Osmose-Lösung getrennt. Die Konzentration der Osmose-Lösung ist größer als die Konzentration der Ausgangslösung. Durch den Konzentrationsgradienten und die semipermeable biomimetische Aquaporin-Membran wandern bevorzugt Wassermoleküle von der Ausgangslösung durch die Aquaporin-Membran hindurch zu der Osmose-Lösung, und zwar ohne Aufbringen von Druck. Die wertgebenden Aroma- und Geschmacksstoffe können die semipermeable biomimetische Aquaporin-Membran nicht passieren und verbleiben somit, ungeachtet ihrer Größe, ihres Molekulargewichts und ihrer chemischen Struktur, in dem Retentat. Durch die Wanderung der Wassermoleküle wird der Ausgangslösung Wasser entzogen, d.h. es entsteht ein Aromastoff-Konzentrat als Retentat, in dem die wertgebenden Aroma- und Geschmacksstoffe aufkonzentriert werden. Die Osmose-Lösung hingegen wird durch die Zuwanderung von Wasser verdünnt; es entsteht ein Permeat. Um das Osmose-System im Gleichgewicht zu halten, wird auf der einen Seite das Lebensmittelkonzentrat kontinuierlich vom System abgezogen und auf der anderen Seite wird frische Osmose-Lösung dem System kontinuierlich zugeführt.

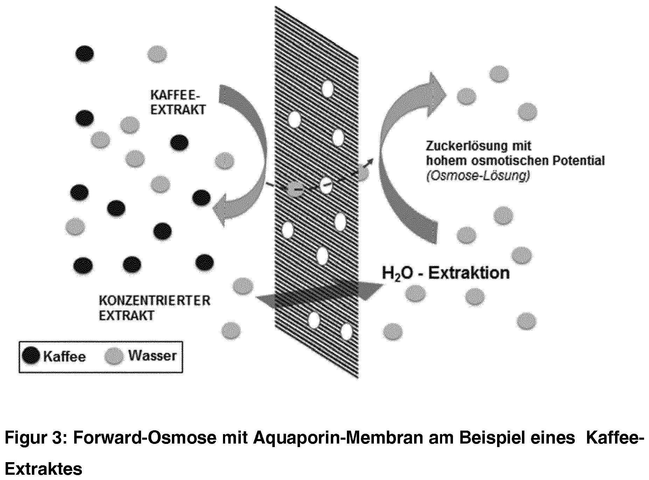

In Figur 3 beispielhaft dargestellt ist die Aufkonzentrierung eines Kaffee-Extraktes (nicht Teil der vorliegenden Erfindung). Ausgangsprodukt ist ein wässriger Kaffee-Extrakt. Diesem Kaffee-Extrakt wird durch Forward-Osmose gegen eine Zuckerlösung mit hohem osmotischen Potential Wasser entzogen. Während des Osmose-Verfahrens verringert sich der Wassergehalt des Kaffee-Extraktes und es entsteht ein konzentrierter Kaffee-Extrakt als Retentat, der von dem System abgezogen und in einem anschließenden Schritt weiterverarbeitet wird, beispielswiese durch Gefriertrocknung. Durch die Selektivität der Aquaporin-Membran für Wasser diffundieren lediglich Wassermoleküle durch die Aquaporin-Membran in Richtung der Zuckerlösung. Die wertgebenden Aroma- und Geschmacksstoffe verbleiben in dem Kaffee-Extrakt und werden angereichert. Die Zuckerlösung wird durch die Zuwanderung von Wasser verdünnt.

Die verwendete biomimetische Membran zeichnet sich aus durch eine hohe Selektivität, welche eine gezielte Abtrennung von Wasser bei gleichzeitigem Rückhalt der wertgebenden Aroma- und Geschmacksstoffe gewährleistet. Dadurch verbleiben die wertgebenden Aroma- und Geschmacksstoffe vollständig in der Wasserphase (Retentat) und wandern nicht über die biomimetische Membran zur Osmose-Lösung. Darüber hinaus ermöglicht die verwendete biomimetische Membran einen hohen Flux und damit eine geringe Verweildauer der Ausgangslösung mit den sensiblen Aroma- und Geschmacksstoffen in der Anlage, was einen Abbau der Aroma- und Geschmacksstoffe, beispielsweise durch Oxidation, verhindert.

Die in dem erfindungsgemäßen Verfahren verwendeten semipermeablen biomimetischen Aquaporin-Membrane kommen entweder als flächige Hohlfaser-Module oder als Spiral-Module zum Einsatz. Bei einem Spiral-Modul ist die Aquaporin-Membran spiralförmig aufgewickelt, wodurch sich die Fläche der Membran erhöhen lässt, was zu einer Verbesserung der Effizienz des Osmose-Vorgangs führt. Vorzugsweise werden in dem Verfahren gemäß der Erfindung Hohlfaser-Module verwendet.

Das erfindungsgemäße Osmose-Verfahren wird bei einem pH-Wert von 2 bis 10 durchgeführt. Darüber hinaus wird das erfindungsgemäße Osmose-Verfahren bei einer Temperatur durchgeführt, die keine negative Auswirkung auf die Inhaltsstoffe, beispielsweise Aroma- und Geschmacksstoffe, der wässrigen Ausgangslösung hat. Das erfindungsgemäße Verfahren wird bei einer Temperatur im Bereich von 10 bis 40 °C durchgeführt, bevorzugt bei einer Temperatur von etwa 25 ° C.

Vorzugsweise wird die Forward-Osmose bei einem Flux, d.h. die Menge Wasser, die durch die Membran strömt, von 4 bis 30 l/m$^2$h, vorzugsweise von 10 bis 20 l/m$^2$h durchgeführt. Besonders bevorzugt wird die Forward-Osmose bei einem Flux von mindestens 12 l/m$^2$h durchgeführt. Hierbei wird bevorzugt drucklos gearbeitet.

Wenn das erfindungsgemäße Verfahren als Umkehrosmose bzw. Reverse-Osmose durchgeführt wird, wird mit einem Druck von 2 bis 15 bar auf die wässrige Ausgangslösung gearbeitet.

In einer weiteren bevorzugten Ausführungsform wird das erfindungsgemäße Osmose-Verfahren kontinuierlich durchgeführt. Dazu wird das durch die MembranFiltration gewonnene Retentat, d.h. das Lebensmittelkonzentrat, aus dem System abgeführt, und im Gegenzug wird das Permeat ebenfalls abgeführt und durch frische Osmose-Lösung ersetzt.

Mit dem erfindungsgemäßen Verfahren wird eine Aufkonzentration der wässrigen Ausgangslösung um den Faktor 20 bis 1.000, vorzugsweise mindestens um den Faktor 100, erzielt.

Die im ersten Schritt des Verfahrens bereitgestellte wässrige Ausgangslösung kann in einem vorgelagerten Schritt durch herkömmliche Verfahrensschritte wie Destillation, Adsorption, Gefriertrocknung, Membranfiltration oder durch Reverse-Osmose oder Forward-Osmose mittels einer Membran aufkonzentriert werden.

Ebenso kann das durch Osmose-Verfahren erhaltene Retentat bzw. Lebensmittelkonzentrat in einem nachgelagerten Schritt durch herkömmliche Verfahrensschritte wie Destillation, Adsorption, Gefriertrocknung, Membranfiltration oder durch Reverse-Osmose oder Forward-Osmose mittels einer Membran weiter aufkonzentriert werden.

Überraschend wurde gefunden, dass das erfindungsgemäße Verfahren zur Aufkonzentrierung eines Brüdenkondensats aus Obst oder Gemüse durch Osmose unter Verwendung der oben beschriebenen biomimetischen Membran zu Aromastoff-Konzentraten führt, deren sensorisches Profil identisch ist mit dem sensorischen Profil des Ausgangsproduktes, d.h. es wird ein Aromastoff-Konzentrat erhalten, das ein authentisches Sensorikprofil aufweist.

Die Analyse der sensorisch wertgebenden Komponenten ergab, dass sowohl flüchtige Geruchs- sowie nichtflüchtige Geschmacksstoffe in den Mengenverhältnissen im Konzentrat wiederzufinden sind, wie sie in der Ausgangslösung vorlagen.

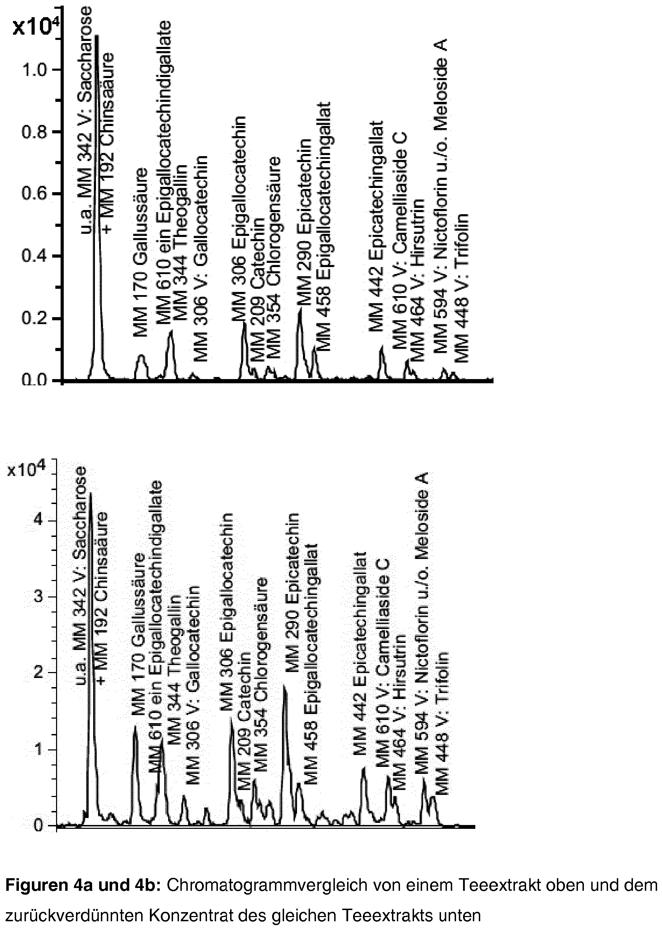

Figur 4 zeigt den flüssigchromatographischen Vergleich nichtflüchtiger Geschmacksstoffe eines wässrigen Teeextraktes und des Konzentrats, welches durch Osmose mittels einer semipermeablen Aquaporin-Membran des wässrigen Teeextraktes gewonnen wurde (nicht erfindungsgemäß). Dazu wurden 600 g schwarzer Tee mit dem 20-fachen seines Gewichts für zwei Stunden mit Wasser extrahiert. Der so erhaltene Extrakt wurde filtriert unter Erhalt der Ausgangslösung. Die Ausgangslösung wurde mittels Forward-Osmose an einem 0,6 m² Aquaporin-Membranmodul im Gegenstrom zu einer Osmose-Lösung (Draw Solution) gefahren. Der Extrakt wurde bei einem Flux von anfänglich 13,5 l/m²h von 1,3 ° Brix auf 33 ° Brix aufkonzentriert. Der Vergleich in Figur 3 zeigt, dass kleine Geschmacksmoleküle wie beispielsweise Einfachzucker, Chinasäure und Gallussäure im Retentat verbleiben und nicht wie bei adsorptiven Verfahren verloren gehen bzw. bei destillativen Verfahren abgebaut werden. Die Flächenverhältnisse der einzelnen wertgebenden Komponenten sind nahezu identisch. Koffein wurde mit > 99 % vollständig im Konzentrat wiedergefunden.

Wasserphasen aus der Fruchtverarbeitung wie beispielsweise von Citrusfrüchten, Beerenfrüchten, Kernobst, Steinfrüchten erfahren während destillativer Aufkonzentrierung eine Veränderung des sensorischen Profils, welche häufig beispielsweise mit "Kochnote", "weniger frisch", "nach Kartoffel", "fettig" und "metallisch" beschrieben wird. Überraschend wurde gefunden, dass beispielsweise Wasserphasen von gelbem Pfirsich und Erdbeere nach Aufkonzentrierung mit einer biomimetischen Membran eine derartige Veränderung des Profils nicht erfahren.

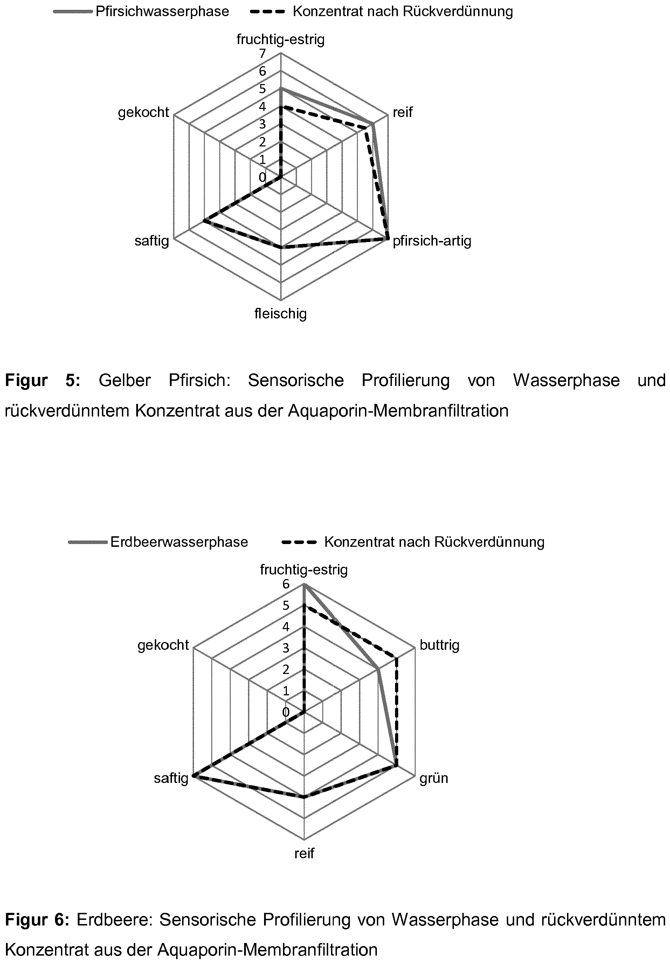

Der sensorische Vergleich anhand eines Sensorikprofils nach DIN10967-1-1999, als Figur 5 zeigt, dass die Pfirsich-Wasserphase und das Konzentrat, welches auf die Anfangskonzentration zurückverdünnt wurde, beinahe identisch riechen. Lediglich eine leicht weniger starke fruchtig-estrige und reife Note wurden wahrgenommen. Die pfirsichartigen und fleischigen Geschmacksnoten, die maßgeblich von schwefelhaltigen Komponenten hervorgerufen werden und bei thermischen sowie adsorptiven Verfahren verändert werden, sind im Konzentrat genauso stark ausgeprägt wie in der Wasserphase.

Zur Erstellung eines sensorischen Profils werden zunächst die beschreibenden Begriffe (Deskriptoren) im Panel gesammelt, wobei die Begriffslisten strukturiert, ähnliche Begriffe zusammengefasst und hedonische Attribute eliminiert werden. Die Bewertung der Intensität der Deskriptoren auf einer Skala von 1 - 10 erfolgt durch mindestens zehn geschulte Prüfpersonen. Dabei werden die Proben codiert, in randomisierter Folge und unter Ausschluss störender Einflüsse wie Farbe, Lärm und Fremdgerüche in einem Sensorikraum verkostet. Die Ermittlung des Endergebnisses erfolgt durch Summierung der Einzelergebnisse und anschließende Bildung des arithmetischen Mittels und wird grafisch in Form eines Netzdiagramms dargestellt.

Tabelle 2: Aromastoffe in der Wasserphase und dem Konzentrat von gelbem Pfirsich

| | Wasserphase [mg/kg] | Konzentrat [mg/kg] | Wieder-findung [%] | Molekular-gewicht [Da] |
|---|---|---|---|---|
| Ethanol | 379,9 | 10295,5 | 48,7 | 46 |
| Ethylacetat | 41,9 | 621,2 | 90,6 | 88 |
| Hexanol | 26,4 | 572,1 | 99,1 | 102 |
| cis-2-Hexenol | 19,6 | 408,4 | 98,1 | 100 |
| trans-2-Hexenal | 9,9 | 128,0 | 98,8 | 98 |
| cis-3-Hexenol | 4,7 | 101,1 | >99,0 | 100 |
| gamma-Decalacton | 2,3 | 33,8 | >99,0 | 170 |
| Hexanal | 1,7 | 31,0 | >99,0 | 100 |
| 3-Methyl-1-butanol | 1,0 | 18,2 | >99,0 | 88 |
| 1,3-Pentenol | 0,3 | 7,7 | >99,0 | 86 |

In Tabelle 2 sind Gehalte von wertgebenden Aromastoffen in der Wasserphase und dem zugehörigen Konzentrat des gelben Pfirsichs angegeben. Die wässrige Ausgangslösung/Wasserphase wurde mittels Destillation aus einem Brüdenkondensat gewonnen, so dass Aromastoffe bereits angereichert wurden. Die weitere Aufkonzentrierung erfolgte mit der biomimetischen Aquaporin-Membran und zeigt, dass bei einem hohen Wasser-Flux von 14 l/m²/h sehr kleine Moleküle mit hoher Ausbeute wiedergefunden werden. Die Wiederfindungsrate der Aromastoffe lag zwischen 90 bis > 99 %. Thermische Abbauprodukte von wertgebenden Bestandteilen konnten nicht nachgewiesen werden. Stattdessen zeigt sich, dass Ethanol entfernt wird, was dem resultierenden Aromastoff-Konzentrat zu Gute kommt. Die Wiederfindungsrate für Ethanol lag bei etwa 50 %.

Die Erdbeere ist ein besonders geeignetes System um negative Einflüsse des Verarbeitungsprozesses auf das sensorische Profil zu untersuchen, da durch thermischen wie druckgetriebenen Einfluss Kochnoten, die den Konsumenten an "gekochte Marmelade" erinnern, entstehen. Die Aufkonzentrierung mittels einer biomimetischen Aquaporin-Membran hat überraschenderweise gezeigt, dass eine Aufkonzentrierung der Geschmackskomponenten ohne negativen Einfluss auf das sensorische Profil bei fast vollständiger Wiederfindung der Geschmackskomponenten und einer hohen Flussrate der Membran möglich ist.

**Tabelle 3:** Aromastoffe in der Wasserphase und dem Konzentrat von Erdbeere

|  | Wasserphase [mg/kg] | Konzentrat [mg/kg] | Wiederfindung [%] | Molekulargewicht [Da] |
|---|---|---|---|---|
| Ethylacetat | 43,1 | 566,8 | 95,0 | 88 |
| trans-2-Hexenal | 37,8 | 540,8 | 99,4 | 98 |
| Hexanol | 28,3 | 543,6 | >99,0 | 102 |
| Methylbutanoat | 18,7 | 245,5 | 99,2 | 102 |
| trans-2-Hexenol | 17,3 | 332,6 | >99,0 | 100 |
| gamma-Decalacton | 12,0 | 127,3 | >99,0 | 170 |
| Ethylbutanoat | 8,4 | 94,3 | >99,0 | 116 |
| Hexansäure | 6,3 | 84,3 | >99,0 | 116 |
| Hexanal | 4,7 | 73,8 | >99,0 | 100 |
| Methylhexanoat | 4,1 | 31,9 | >99,0 | 130 |
| 2-Methylbuttersäure | 4,0 | 25,0 | >99,0 | 102 |
| Ethylhexanoat | 1,7 | 7,1 | >99,0 | 144 |
| Methylpropionat | 1,3 | 14,2 | >99,0 | 88 |
| Butylacetat | 1,1 | 13,2 | >99,0 | 116 |
| Propylacetat | 0,7 | 7,2 | >99,0 | 102 |
| Methylethylacetat | 0,6 | 11,0 | >99,0 | 102 |

In Tabelle 3 sind Gehalte von den Geschmackskomponenten in der Wasserphase und dem zugehörigen Konzentrat der Erdbeere angegeben. Ein Anreicherungsfaktor von 19 wurde bei hohem Wasser-Flux in kurzer Zeit erreicht. Die Wiederfindungsrate der Aromastoffe lag zwischen 95 bis > 99 %.

Der sensorische Vergleich in Figur 6 zeigt, dass die wässrige Ausgangslösung/Wasserphase und das Konzentrat, welches auf die Anfangskonzentration zurückverdünnt wurde, beinahe identisch riechen. Lediglich eine leicht weniger starke fruchtig-estrige Note bei leicht erhöhtem Eindruck einer buttrigen Note wurde wahrgenommen.

Die Aufkonzentrierung von Gemüsesäften wie beispielsweise von Tomate, Gurke, Karotte, Sellerie, rote Beete und Zwiebel führt mit herkömmlichen, aus dem Stand der Technik bekannten Prozessen ebenfalls zu einer unerwünschten Veränderung des sensorischen Profils. Überraschend wurde gefunden, dass das erfindungsgemäße Verfahren zu einer Aufkonzentrierung ohne die besagten negativen Einflüsse führt. Beispielsweise wurden 20 kg Gurkensaft aus 75 geschälten und pürierten Salatgurken nach Dekantieren, Zentrifugation und Sackfiltration bei einer Maschenweite von 100 μm gewonnen und mittels Umkehrosmose bzw. dem hierin beschriebenen Osmose-Verfahren aufkonzentriert. Dazu wurden 10 kg des Gurkenwassers zuerst bei Raumtemperatur und 10 bar mit einem herkömmlichen 0,37 m² Reverse-Osmose-Membranmodul von General Electric 2,5 Stunden lang bei einem Druck von 30 bar und weiteren 3,5 Stunden bei einem anfänglichen Fluss von 4 l/m²/h bis zu einer Restmenge von 1640 g entwässert. Die zweite Hälfte 10 kg Gurkenwasser wurde mit einem 0,6 m² Aquaporin-Membranmodul gegen 2 kg eine Kaliumlactat-Lösung als Draw Solution mit einer Konzentration von 60 ° Brix bei 300 ml/min für 5 Stunden gefahren. Dabei wurden 1.091 g Produkt ausgewogen. Der Membran-Flux betrug anfangs 12 l/m²/h. Das hierin beschriebene Osmose-Verfahren mit der biomimetischen Aquaporin-Membran wies einen 3-fach höheren anfänglichen Membran-Flux auf und konzentrierte das Gurkenwasser in der gleichen Zeit deutlich stärker auf als die angewandte konventionelle Umkehrosmose. (Das Aufkonzentrieren von Säften und Extrakten aus Obst oder Gemüse mit Aquaporin-Membranen, ohne ein Brüdenkondensat im Verfahren zu erhalten, ist nicht Teil der vorliegenden Erfindung.)

**Tabelle 4:** Aromastoffe in Gurkenwasser und dem Konzentrat aus Umkehrosmose und Aquaporin-Membran-Osmose

|  | Gurkenwasser [mg/kg] | Umkehrosmose [mg/kg] | AQP-Membran-Osmose [mg/kg] |
|---|---|---|---|
| 6-(Z)-Nonenol | 0,600 | 2,823 | 0,148 |
| 2,6-(E,Z)-Nonadienol | 0,521 | 0,800 | 0,104 |
| Hexanol | 0,210 | 1,085 | 0,138 |

(fortgesetzt)

| | Gurkenwasser [mg/kg] | Umkehrosmose [mg/kg] | AQP-Membran-Osmose [mg/kg] |
|---|---|---|---|
| 2,6-(E,Z)-Nonadienal | 0,078 | 0,886 | 2,610 |
| 2-(E)-Nonenal | 0,039 | 0,428 | 0,715 |
| 8,11,14-(Z, Z, Z)-Heptadecatrienal | 0,025 | 0,247 | 0,769 |
| 2-(E)-Hexenal | 0,007 | 0,078 | 0,296 |
| Hexanal | 0,003 | 0,074 | 0,773 |
| 2,6-(E,E)-Nonadienal | <0,002 | 0,035 | 0,042 |
| 2-(E)-Octenal | <0,002 | <0,002 | 0,039 |
| 2,4-(E,E)-Decadienal | <0,002 | <0,002 | 0,061 |
| 8,11-(Z,Z)-Heptadecadienal | <0,002 | <0,002 | 0,604 |

Die Umkehrosmose gilt industriell als das Standardverfahren zur nichtthermischen und damit schonenden Aufkonzentrierung durch Entwässerung des eingesetzten Saftes/der wässrigen Ausgangslösung.

Der in Tabelle 4 aufgeführte Vergleich zeigt allerdings, dass die wertgebenden Aldehyde in Gurke wie 2,6-(E,Z)-Nonadienal und 2-(E)-Hexenal durch Umkehrosmose abgebaut und Alkohole gebildet werden, während bei der Osmose mittels der biomimetischen Aquaporin-Membran die Aldehyde mengenmäßig dominieren und somit erhalten bleiben. Der Versuch hat überraschend gezeigt, dass das Konzentrat aus dem hierin beschriebenen Verfahren einen intensiven Geruch nach frisch angeschnittener Gurke hatte und das bereits bräunlich gefärbte Konzentrat aus der Umkehrosmose grünlich, balsamisch, nach gekochter Gurke roch. Zudem war die Draw Solution der Osmose mit der biomimetischen Aquaporin-Membran geruchslos, während das Permeat der Umkehrosmose leichte Grünnoten aufwies. Weitere Osmose-Versuche mit klassischen, aus dem Stand der Technik bekannten Membranen zeigten wie bei der Umkehrosmose, dass mit klassischen Membranen Verluste an Geschmackskomponenten auftreten. Die verwendete biomimetische Membran gemäß dem hierin beschriebenen Verfahren zeigt demnach eine hohe Selektivität, welche eine gezielte Abtrennung von Wasser bei Rückhaltung der wertgebenden Geschmackskomponenten bei gleichzeitig hohem Flux und damit geringer Verweildauer der sensiblen Geschmackskomponenten in der Anlage ermöglicht.

Überraschenderweise wurden bei den beschriebenen Versuchen keine Abbauprodukte oder störende Abbauprodukte der wertgebenden Komponenten nachgewiesen und sensorisch störende Komponenten wurden ebenfalls nicht detektiert. Bei den Störkomponenten handelt es sich insbesondere um solche Verbindungen, die bei destillativen Aufkonzentrationsverfahren nach dem Stand der Technik beispielsweise durch thermischen Abbau, durch pH-Wert-Verschiebung, Oxidation oder durch chemische Umlagerungsreaktionen gebildet werden, und das sensorische Profil des Aromastoff-Konzentrates beeinflussen.

Aromastoffe, die je nach Lebensmittel und Konzentration das Aromaprofil negativ beeinflussen und das Ergebnis eines prozessbedingten physikalischen und chemischen Abbaus sind, sind in der nachfolgenden Tabelle 5 aufgeführt:

Tabelle 5: Einfluss des Membranprozesses auf Geruchs- und Geschmackskomponenten

| Lebensmittel | Prozessbedingter Verlust | Bildung Störkomponente(n) |
|---|---|---|
| Ananas | 4-Hydroxy-2,5-dimethyl-3(2H)-furanon, Ethyl-2-methylbutanoat | Methional, 2,4-(E,E)-Decadienal |
| Apfel | 2-(E)-Hexenal, cis-3-Hexenol, Hexanal, Ethylbutanoat, 2-Methylbutylacetat | 2-3-Methylbutyraldehyd, Furfural, 1,3-Pentanal, 3,5-Octadienal, Linalooloxid, 8-p-Cymenol |
| Bier | Isoamylacetat, 3-Methylbutanol, Linalool, Myrcen, Phenylethanol, Ethylbutanoat | trans-2-Nonenal, Dimethylsulfid |
| Citrusfrüchte | Acetaldehyde, cis-3-Hexenal, Hexanal, Ethylbutanoat | $\alpha$-Terpineol, p-Dimethylstyrol, Limonenepoxid, Carveol, Carvon, 4-Vinylguaiacol |
| Erdbeere | Acetaldehyde, Methylbutanoat, Ethyl-2-methylbutanoat, cis-3-Hexenal, Hexanal | $\beta$-Damascenon, 2,4-(E,E)-Decadienal, Furfural, Guajacol |
| Kaffee | Methanthiol, Dimethylsulfide, Methylpropanal, 2,3-Butandione, 2-Furfurylthiol | Bis-(2-furfuryl)disulfid, 2-(2-furyl)Methylthio-hydroxy-1,4-dihydro-pyrazin |

(fortgesetzt)

| Lebensmittel | Prozessbedingter Verlust | Bildung Störkomponente(n) |
|---|---|---|
| Milch | Diacetyl, 2-Methylbutanol, cis-4-Heptenal, trans-2-Nonenal, 1-Octen-3-one, 1-Octen-3-ol | Dimethylsulfid, Dimethyldisulfid, 2-Hexanon, 2-Heptanon, 2,3-Methylbutanal |
| Passionsfrucht | Acetaldehyde, Hexanal, Ethylbutyrate, Linalool | Benzaldehyd, $\alpha$-Terpineol, Furfural, Essigsäure, Acetophenon, $\beta$-Ionon, Linalooloxid |
| Tee | cis-3-Hexenal, cis-3-Heptenal, Linalool, 3-Methyl-2,4-nonandion, 2,3-Methylbuttersäure, 1,5-Octadien-3-on | $\beta$-Damascenon, 2,4-(E,E)-Decadienal, 3-Hydroxy-4,5-dimethyl-2(5H)-furanon, 4-Hydroxy-2,5-dimethyl-3(2H)-furanon |
| Tomate | cis-3-Hexenol, cis-3-Hexenal, trans-2-Hexenal, Hexanal, $\beta$-Ionon, 2-Isobutylthiazol | Dimethyldisulfid, Methional, 4-Hydroxy-2,5-dimethyl-3(2H)-furanon |
| Zwiebelsaft | Propanal, Hexanal, trans-2-Heptenal, Dipropyldisulfid, Dipropyltrisulfid, Methylpropyldisulfid | Dimethyltrisulfid, Dipropyltrisulfid, Dimethyltetrasulfid, 3,5-Diethyl-1,2,4-trithiolan, Thiophen |

Eine Aufkonzentrierung der in Tabelle 5 genannten Lebensmittel mit dem erfindungsgemäß beschriebenen Verfahren führt zu einem deutlich authentischeren sensorischen Profil, als mit herkömmlichen, aus dem Stand der Technik bekannten Destillations-, Adsorptions- und Membranverfahren. Insbesondere entstehen keine prozessbedingten Verluste an Aromastoffen und es werden keine Störkomponenten gebildet, die das sensorische Profil verändern.

Ein weiterer Vorteil des erfindungsgemäßen Verfahrens ist, dass es im Vergleich zu herkömmlichen thermischen, beispielsweise destillativen Verfahren, zur Aufkonzentrierung für thermolabile Lebensmittel bzw. Lebensmittel mit thermolabilen Inhaltsstoffen besonders geeignet ist, da es bei niedrigen Temperaturen, vorzugsweise bei 25°C, durchgeführt werden kann und somit Aromastoff-Konzentrate erhalten werden können, deren Geschmack und Aroma nicht durch Hitze beeinflusst wird.

Die Aromastoff-Konzentrate, die durch das erfindungsgemäße Verfahren gewonnen werden, zeichnen sich auch dadurch aus, dass sie keine Lösungsmittelzusätze enthalten. Im Unterschied zu den herkömmlichen, aus dem Stand der Technik bekannten Adsorptionsverfahren zur Aufkonzentrierung von wässrigen Ausgangslösungen aus einem Lebensmittel werden im erfindungsgemäßen Verfahren keine Lösungsmittel verwendet; somit sind keine Lösungsmittel-Rückstände im erhaltenen Aromastoff-Konzentrat vorzufinden. Damit werden gleichzeitig auch Lösungsmittel-bedingte Ausfällungsreaktionen und Verfärbungen vermieden, so dass klare, nicht getrübte Aromastoff-Konzentrate erhalten werden. Lösungsmittel, die typischer Weise in solchen Adsorptionsverfahren zur Gewinnung des Aromakonzentrates aus Lebensmitteln verwendet werden, sind Aceton, Butan, Butan-1-ol, Butan-2-ol, Cyclohexan, Distickstoffmonoxid, Diethylether, Ethylacetat, Ethanol, Ethylmethylketon, Hexan, Methanol, Methylacetat, Propan, Propan-1-ol und Propan-2-ol und die nicht originär aus dem Lebensmittel stammen.

Die vorliegende Erfindung betrifft somit auch ein Aromastoff-Konzentrat, welches nach dem zuvor beschriebenen Verfahren erhältlich ist.

Gemäß der Erfindung ist das Lebensmittel-Konzentrat ein Aromastoff-Konzentrat.

Wie oben ausführlich beschrieben, weist das erfindungsgemäße Aromastoff-Konzentrat kein Off-flavour auf. Off-flavour ist die anerkannte und verwendete Fachbezeichnung für Aromafehler. Aromafehler entstehen dadurch, dass in den betroffenen Lebensmitteln eine sonst nicht vorkommende Aromakomponente zusätzlich vorhanden ist, eine oder mehrere charakteristische Verbindung(en) (impact compounds) verloren gingen oder eine Konzentrationsverschiebung zwischen den einzelnen Aromakomponenten stattgefunden hat.

Wie aus der obigen detaillierten Beschreibung ersichtlich ist, betrifft die vorliegende Erfindung auch ein anspruchsgemäßes Aromastoff-Konzentrat, bei dem das sensorische Profil eines rückverdünnten Konzentrats aus der erfindungsgemäßen Osmose mit der semipermeablen biomimetischen Membran von dem sensorischen Profil einer wässrigen Ausgangslösung bei einer Skala von 0 bis 10 Punkten in den 6 Geschmacksachsen eines konventionelles Panel-Profils um insgesamt max. 1 Punkt abweicht, wobei das sensorische Profil über ein Testpanel nach DIN 10967-1-1999 gemessen wird, bzw. nach dem im Folgenden beschriebenen erfindungsgemäßen Protokoll.

Zur Erstellung eines sensorischen Profils werden zunächst die beschreibenden Begriffe (Deskriptoren) im Panel gesammelt, wobei die Begriffslisten strukturiert, ähnliche Begriffe zusammengefasst und hedonische Attribute eliminiert werden. Die Bewertung der Intensität der Deskriptoren auf einer Skala von 1 - 10 erfolgt durch mindestens zehn geschulte Prüfpersonen. Dabei werden die Proben codiert, in randomisierter Folge und unter Ausschluss störender Einflüsse wie Farbe, Lärm und Fremdgerüche in einem Sensorikraum verkostet. Die Ermittlung des Endergebnisses erfolgt durch Summierung der Einzelergebnisse und anschließende Bildung des arithmetischen Mittels und wird grafisch in Form eines Netzdiagramms dargestellt.

Wie aus den Figuren 5 und 6 ersichtlich ist, weicht das sensorische Profil des Konzentrats nach Rückverdünnung, welches nach dem beschriebenen Verfahren gewonnen wurde, von dem sensorischen Profil der wässrigen Ausgangslösung (Wasserphase) von gelbem Pfirsich und Erdbeere in den entsprechenden 6 Geschmacksachsen jeweils um max. 1 Punkt voneinander ab.

Wie weiter anhand der obigen Analysen ersichtlich ist, ist bei dem erfindungsgemäßen Aromastoff-Konzentrat die Wiederfindungsrate der Aromastoffe, welche in der wässrigen Ausgangslösung einen OAV (odor activity value) ≥ 1 haben, in dem Retentat > 90 %, bevorzugt > 98 %, insbesondere > 99 %, bezogen auf die wässrige Ausgangslösung. Wie aus den Tabellen 3 und 5 eindeutig hervorgeht, führt die erfindungsgemäße Aufkonzentrierung der jeweiligen Brüdenkondensate aus Obst oder Gemüse zu einer Aufkonzentrierung der Geschmackskomponenten ohne negativen Einfluss auf das sensorische Profil bei fast vollständiger Wiederfindung der Geschmackskomponenten, wodurch ein authentisches sensorisches Profil erhalten wird.

Der OAV-Wert wird definiert als Maßstab der Bedeutung einer spezifischen Komponente an dem Geruch einer Probe, beispielsweise eines Lebensmittels. Der OAV-Wert wird berechnet durch Division der Konzentration einer bestimmten Substanz im Lebensmittel (mg/kg) durch den Konzentrationsschwellenwert dieser Substanz im Lebensmittel (mg/kg). Bei einem OAV > 1 überschreitet die Konzentration des Stoffes die Geruchsschwellen-Konzentration und die Substanz leistet dann einen sensorischen Beitrag zum Gesamtprofil. Substanzen mit einem OAV < 1 tragen somit zum sensorischen Profil nicht bei.

Mit dem hierin beschriebenen Verfahren können zudem Konzentrate bereitgestellt werden, die sich besonders hervorheben. Diese zeichnen sich durch höhere Authentizität im Geschmack und Geruch aus, da bei dem Verfahren geruchsaktive Substanzen erhalten bleiben. Es wurde festgestellt, dass die Aromastoff-Konzentrate durch die folgende Beziehung dargestellt werden können: das erfindungsgemäße Aromastoff-Konzentrat ist demnach dadurch gekennzeichnet, dass die Aromastoffe, d.h. die geruchsaktiven Substanzen, in dem Aromastoff-Konzentrat mit einem Parameter W (log des Sensorischen Wiederfindungskoeffizienten), der vorzugsweise > - 3, noch bevorzugter > - 5 ist, bezogen auf die wässrige Ausgangslösung, eine Wiederfindungsrate von ≥ 50 %, bevorzugt von ≥ 70 %, hat. Besonders bevorzugt ist hierbei eine Wiederfindungsrate von ≥ 90 % oder ≥ 95 %, und am bevorzugtesten eine Wiederfindungsrate von ≥ 98 % oder ≥ 99 %.

Der Sensorische Wiederfindungskoeffizient (SWF) eines Aromastoffs setzt sich zusammen aus dem logP des Aromastoffs und der Geruchsschwellen-Konzentration des Aromastoffs (mg/kg in Wasser) und ist wie folgt definiert:

$$\text{Sensor. Wiederfindungskoeffizient SWF} = \frac{10^{\wedge}\text{logP}}{\text{Geruchsschwellen}-\text{Konzentration} \left(\frac{\text{mg}}{\text{kg}}\right)}$$

Der logP-Wert wiederum ist wie folgt definiert:

$$\text{logP} = \log K(\text{n-Octanol/Wasser}) = \frac{\text{Cn}-\text{Octanol}}{\text{CWasser}},$$

worin:

C (n-Octanol) die Konzentration des Aromastoffes in der n-Octanol-Phase ist; und
C (Wasser) die Konzentration des Aromastoffes in der Wasser-Phase ist.

Der log K(n-Octanol/Wasser) ist ein dimensionsloser Verteilungskoeffizient, der das Verhältnis der Konzentration des Aromastoffs in einem Zweiphasensystem aus n-Octanol und Wasser angibt. Er ist somit ein Modellmaß für die Polarität bzw. Wasser-/Fettlöslichkeit des Aromastoffes.

LogP ist ein gängiger physikalischer Parameter und ist positiv bei liphophilen Aromastoffen und negativ bei hydrophilen Aromastoffen.

Der W-Parameter ist somit wie folgt definiert:

$$\text{W} = log_{10}(SWF)$$

Der W-Parameter ist somit eine Absolut-Größe des jeweiligen Stoffes, der unter Berücksichtigung der sensorischen Aktivität der Substanzen eine Diskriminierung gemäß der Erfindung gestattet.

Die logP, Geruchsschwellen-Werte und sensorischen Wiederfindungskoeffizienten von Aromastoffen sind in der nachfolgenden Tabelle 6 wiedergegeben:

## Tabelle 6: Geruchsschwellen-Werte und Sensorische Wiederfindungskoeffizienten

| Verbindung | logP | Schwelle [mg/kg in Wasser] | Sensorischer Wiederfindungs-koeffizient (SWF) | W = log (SWF) |
|---|---|---|---|---|
| Methanol | -0,358 | 369828 | 1,18577E-06 | -5,93 |
| Ethanol | -0,19 | 52000 | 1,24164E-05 | -4,91 |
| Propanol | 0,5145 | 5700 | 0,000573621 | -3,24 |
| Hexanol | 1,8831 | 50 | 1,528023367 | 0,18 |
| Z-3-Hexenol | 1,4385 | 13 | 2,111332572 | 0,32 |
| Acetaldehyd | -0,1825 | 63 | 0,010427002 | -1,98 |
| Propanal | 0,4844 | 170 | 0,017945315 | -1,75 |
| 2-Methylbutyraldehyd | 1,4031 | 0,9 | 28,10978284 | 1,45 |
| Hexanal | 1,853 | 16 | 4,455331438 | 0,65 |
| Essigsäure | -0,2299 | 180000 | 3,27211E-06 | -5,48 |
| Buttersäure | 0,8932 | 1000 | 0,007819878 | -2,11 |
| Hexansäure | 1,8056 | 3944 | 0,016205525 | -1,79 |
| Ethylacetat | 0,73 | 3125 | 0,001718502 | -2,76 |
| Ethylbutanoat | 1,493 | 1 | 31,11716337 | 1,49 |
| Ethylhexanoat | 2,4054 | 5 | 50,86628212 | 1,71 |
| 2,3-Butandion | -0,5078 | 54 | 0,005751833 | -2,24 |
| 2,3-Pentandion | 0,1591 | 5 | 0,28848949 | -0,54 |
| gamma-Octalacton | 2,0901 | 24 | 5,127300347 | 0,71 |
| Linalool | 2,7354 | 103 | 5,279135088 | 0,72 |
| Geranial | 3,1904 | 320 | 4,84451184 | 0,68 |

Geruchsaktive Substanzen, mit einem log des Sensorischen Wiederfindungskoeffizienten W der Aromastoffe > - 3 werden, ungeachtet ihrer Größe, ihres Molekulargewichts und ihrer chemischen Struktur, durch die biomimetische Membran gut in dem Retentat zurückgehalten, weswegen die erhaltenen Konzentrate ein sehr authentisches und nahezu deckungsgleiches sensorisches Profil, bezogen auf die wässrige Ausgangslösung, aufweisen. Methanol und Ethanol wandern aufgrund ihrer polaren Struktur partiell durch die Aquaporin-Wasserkanäle hindurch; allerdings sind diese Stoffe aufgrund ihres hohen Schwellenwertes nur von geringer Relevanz für das sensorische Profil.

Besonders bevorzugt ist der Erfindung nach ein Aromastoff-Konzentrat bei dem der Ethanol-Gehalt um mindestens 50 % reduziert ist, bezogen auf die wässrige Ausgangslösung. Vorteilhaft an dem erfindungsgemäßen Verfahren ist demnach eine starke Reduzierung von Ethanol, bei gleichzeitig starker Zurückhaltung der charakteristischen Aromastoffe. Damit lassen sich Konzentrate auch in Ländern verkaufen, die kulturell-bedingte Vorbehalte gegenüber Ethanol haben. Noch bevorzugter ist die Wiederfindungsrate von Ethanol nach dem vorliegenden Verfahren < 90 %, noch bevorzugter < 99 %, sodass die Ethanolanteile signifikant reduziert werden können, ohne dass der charakteristische Geschmack der Ausgangslösung verloren geht.

Bevorzugt ist die Wiederfindungsrate der Aroma- und Geschmacksstoffe mit einem Molekulargewicht von 40 bis 300 Da, vorzugsweise von 86 bis 170 Da, in dem Retentat > 90 % ist, bezogen auf die wässrige Ausgangslösung, noch bevorzugter die Wiederfindungsrate der Aromastoffe mit einem Molekulargewicht von 98 bis 200 Da > 98 %.

Ein weiterer besonderer Vorteil des erfindungsgemäßen Aromastoff-Konzentrates ist, dass es frei ist von Lösungsmittelzusätzen, insbesondere frei ist von Lösungsmittelzusätzen, die ausgewählt sind aus der Gruppe, die besteht aus: Aceton, Butan, Butan-1-ol, Butan-2-ol, Cyclohexan, Distickstoffmonoxid, Diethylether, Ethylacetat, Ethanol, Ethylmethylketon, Hexan, Methanol, Methylacetat, Propan, Propan-1-ol und Propan-2-ol. Bei den o.g. Lösungsmittelzusätzen handelt es sich um solche Lösungsmittel, die bei den herkömmlichen adsorptiven Verfahren zur Elution/Desorption der wertgebenden Aroma- und Geschmacksstoffe und damit zur Gewinnung des Aromakonzentrates eingesetzt werden und die nicht originär aus dem nativen Lebensmittel stammen. Diese Lösungsmittel verbleiben als Rückstände in dem Aromakonzentrat und sind teilweise toxikologisch bedenklich.

Eine vorteilhafte Eigenschaft des erfindungsgemäßen Aromastoff-Konzentrats ist auch, dass es frei von störenden Aromakomponenten ist, bevorzugt, dass das Aromastoff-Konzentrat frei von störendenden Aromakomponenten mit einem OAV (odor activity value) ≥ 1 ist, welche das sensorische Profil verändern.

Bei den Störkomponenten handelt es sich insbesondere um solche Verbindungen, die bei herkömmlichen Osmoseverfahren mit bekannten Membranen oder bei destillativen Verfahren zur Gewinnung des Aromakonzentrates, beispielsweise durch thermischen Abbau, durch pH-Wert-Verschiebung, durch Oxidation oder durch chemische Umlagerungsreaktionen, gebildet werden und somit das sensorische Profil des Lebensmittelkonzentrates beeinflussen. Solche durch thermischen Abbau von Lebensmittel-Inhaltsstoffen, beispielsweise bei den herkömmlichen destillativen Verfahren zur Gewinnung des Aromakonzentrates gebildeten Störkomponenten können das sensorische Profil des erhaltenen Aromastoff-Konzentrates negativ verändern.

Derartige Störkomponenten sind vorzugsweise:

| Bei Ananas: | Methional, 2,4-(E,E)-Decadienal |
|---|---|
| Bei Apfel: | 2,3-Methylbutyraldehyd, Furfural, 1,3-Pentanal, 3,5-Octadienal, Linalooloxid, 8-p-Cymenol |
| Bei Citrusfrüchten: | $\alpha$-Terpineol, p-Dimethylstyrol, Limonenepoxid, Carveol, Carvon, 4-Vinylguaiacol |
| Bei Erdbeere: | $\beta$-Damascenon, 2,4-(E,E)-Decadienal, Furfural, Guajacol |
| Bei Passionsfrucht: | Benzaldehyd, $\alpha$-Terpineol, Furfural, Essigsäure, Acetophenon, $\beta$-Ionon, Linalooloxid |
| Bei Tomate: | Dimethyldisulfid, Methional, 4-Hydroxy-2,5-dimethyl-3(2H)-furanon |
| Bei Zwiebelsaft: | Dimethyltrisulfid, Dipropyltrisulfid, Dimethyltetrasulfid, 3,5-Diethyl-1,2,4-trithiolan, Thiophen |

In einer weiter bevorzugten Ausführungsform ist das erfindungsgemäße Aromastoff-Konzentrat frei von störenden Aromakomponenten mit einem OAV (odor activity value) ≥ 1 und/oder enthält keine Lösungsmittelzusätze. Selbst bei einer mindestens 100-fachen Aufkonzentration, insbesondere einer 200 bis 1.000-fachen Aufkonzentration ist das erfindungsgemäße Aromastoff-Konzentrat frei von störenden Aromakomponenten mit einem OAV (odor activity value) ≥ 1 und/oder enthält keine Lösungsmittelzusätze. Demnach ist das erfindungsgemäße Aromastoff-Konzentrat mindestens 100-fold, insbesondere 200- bis 1.000-fold.

Bei diesem Aromastoff-Konzentrat, sind die Störkomponenten vorzugsweise wie oben definiert und, je nach Ausgangslösung, sind für die jeweiligen Lebensmittel-Ausgangslösungen keine der oben definierten Störkomponenten in solchen Mengen enthalten, die geruchsaktiv sind. Weiterhin sind keine Lösungsmittelzusätze enthalten, die üblicherweise bei adsorptiven Aufkonzentrationsverfahren als Elutionsmittel eingesetzt werden. Dieses Aromastoff-Konzentrat ist somit einzigartig in Geschmack und Zusammensetzung bei diesen Konzentrationsverhältnissen, z.B. bei einer Konzentration der Aromastoffe von 1000 bis 10000 ppm. Diese Lebensmittelkonzentrate haben generell eine geringere Bräune und weniger Ausfällungsreaktionen.

Das erfindungsgemäße Aromastoff-Konzentrat ist vorzugsweise ein Konzentrat, welches aus einem oder mehreren der folgenden Lebensmittel hergestellt wird: Fruchtsäfte wie Ananassaft, Apfelsaft, Aroniasaft, Citrusfrüchte-Saft, Erdbeersaft, Passionsfruchtsaft und Birnensaft; Gemüsesäfte wie Gurkensaft, Karottensaft, Spargelsaft, Tomatensaft, Zwiebelsaft; Presssäfte von Basilikum, Kiwi, Mango, Petersilie, Sellerie, oder Spinat.

Das erfindungsgemäße Aromastoff-Konzentrat kann zur Aromatisierung oder Rekonstitution des Aromas von Lebensmitteln, Getränkeprodukten, Halbfertigprodukten, Mundhygiene-produkten, kosmetischen oder pharmazeutischen Produkten verwendet werden.

**Beispiele**

Das Verfahren der vorliegenden Erfindung und die damit erhaltenen Aromastoff-Konzentrate werden nun anhand einzelner Beispiele näher beschrieben.

Im Allgemeinen werden die folgenden Durchführungsparameter verwendet, wenn nicht explizit in den Beispielen anders aufgeführt:

Verfahrenstechnische Details zum Betrieb des Aquaporin-Membranmoduls:

- Moduldesign: Rohr aus Polycarbonat, 23 cm lang und 5 cm Durchmesser, durchzogen mit Hohlfasern (sog. Hohlfasermodul)
- Flux: mind. > 12 l/m$^2$/h
- pH: 2 bis 11
- Temperatur: max. 50 °C
- Drucklos bei Fluss von max. 18 l/h der Wasserphase und max. 18 l/h der Osmose-Lösung bei einem 0,6 m$^2$ Modul
- 4 M NaCl als Osmose-Lösung

**Beispiel 1 (nicht erfindungsgemäß): Herstellung eines Gurkensaft-Konzentrates**

| | | |
|---|---|---|
| Produkt: | Gurkensaft, frisch entsaftet | |
| Einsatz: | 10.000 g | Gurkensaft, Wasserphase |
| | 2.000 g | Osmoselösung 60 ° Brix Kaliumlactat |

Konzentrierung der Aromastoffe um den Faktor 20

| | |
|---|---|
| Apparatur: | Forward-Osmose-Modul von Aquaporin LHF033 kombiniert mit 10 Liter-Tank von Sartorius + 2 Ismatec Pumpen |
| Membran: | FO Modul Aquaporin LHF033, 0,6m$^2$ aktive Membranfläche |
| Versuchsdurchführung: | Der Gurkensaft sowie die Draw Solution wurden wegen enthaltener Fest- bzw. Schwebstoffe über 100 μm Sackfilter vorfiltriert. Der Feed-Tank wurde mit 10 kg Gurkensaft befüllt und 2,0 kg Draw Solution (Osmose-Lösung) im Kanister vorgegeben. Danach wurde die Feed-Pumpe und die Draw Solution-Pumpe mit jeweils einem Fluss von 300 ml/min gestartet. Nach Abnahme von 9 kg wird der Versuch beendet. Der Feed Tank wird mit 2 kg destilliertem Wasser nachgespült. Das Spülwasser wird ebenfalls zur Analytik gegeben. |

Auswaagen:

| | |
|---|---|
| Ausgang: | 10.000 g |
| Permeat: | 8.835 g |
| Retentat: | 1.091 g |
| Spülwasser (Auswaage): | 2.004 g |

Draw Solution Start: 60 ° Brix
Draw Solution Ende: 12,4 ° Brix

Temperatur: 25°C

Aufzeichnung der Daten:

| Zeit (min) | 0 | 15 | 30 | 60 | 90 | 120 | 150 |
|---|---|---|---|---|---|---|---|
| Menge des zugesetzten Produktes (g) | 10.00 0 | | | | | | |
| Menge der zugesetzten Draw Solution (kg) | 2,0 | | | | | | |
| Gewicht Draw Solution (kg) | 0 | 2.013 | 3.352 | 5.150 | 6.352 | 7.227 | 7.912 |
| Flux (l/m$^2$·h) | | | 11,7 | 8,6 | 7,1 | 6,0 | 5,3 |

| Zeit (min) | 180 | 210 | 240 | 270 | 300 |
|---|---|---|---|---|---|
| Gewicht Draw Solution (kg) | 8.385 | 8.629 | 8.753 | 8.810 | 8.835 |
| Flux (l/m$^2$·h) | 4,7 | 4,1 | 3,6 | 3,3 | 2,9 |

**Beispiel 2 (nicht erfindungsgemäß): Herstellung eines Gelben Pfirsich-Konzentrates**

| | | |
|---|---|---|
| Produkt: | Gelber Pfirsich, reiner Extrakt | |
| Einsatz: | 10.000 g | Gelber Pfirsich, Wasserphase |
| | 2.000 g | Osmoselösung 60 ° Brix Kaliumlactat |

Konzentrierung der Aromastoffe um den Faktor 20

| | |
|---|---|
| Apparatur: | Forward-Osmose-Modul von Aquaporin LHF033 kombiniert mit 10 Liter-Tank von Sartorius + 2 Ismatec Pumpen |
| Membran: | FO Modul Aquaporin LHF033, 0,6m$^2$ aktive Membranfläche |
| Versuchsdurchführung: | Der Feed-Tank wurde mit 10.000g Wasserphase befüllt. 2,0 kg Draw Solution (60 ° Brix) im Kanister vorgegeben. Danach wurde die Feed-Pumpe und die Draw Solution-Pumpe mit jeweils einem Fluss von 300 ml/min gestartet. |

Auswaagen:

| | |
|---|---|
| Ausgang: | 10.000 g |
| Retentat: | 413,5 g |
| Permeat: | 9.500 g |

Draw Solution Start: 60 ° Brix
Draw Solution Ende: 12,6 ° Brix
Temperatur: 25°C

Aufzeichnung der Daten:

| Zeit (min) | 0 | 15 | 30 | 60 | 116 Ende |
|---|---|---|---|---|---|
| Menge des zugesetzten Produktes (g) | 10.000 | | | | |
| Menge der zugesetzten Draw Solution (kg) | 2,0 | | | | |
| Gewicht Draw Solution (kg) | 0 | 2.100 | 3.890 | 6.500 | 9.500 |
| Flux (l/m$^2$·h) | | 14,0 | 11,9 | 8,7 | 5,4 |

Das sensorische Profil ist wie folgt:

Intensität: 1 - 10

| | Ausgang Gelber Pfirsich, Wasserphase | Konzentrat nach Rückverdünnung |
|---|---|---|
| Fruchtig-estrig | 5 | 4 |
| Reif | 6 | 5,5 |
| Pfirsich-artig | 7 | 7 |
| Fleischig | 4 | 4 |
| Saftig | 5 | 5 |
| Gekocht | 0 | 0 |

Das sensorische Profil ist in Figur 5 wiedergegeben.

**Beispiel 3: Herstellung eines Erdbeer-Konzentrates**

| Produkt: | Wasserphase aus der Erdbeersaftkonzentrierung | |
|---|---|---|
| Einsatz: | 10.000 g | Erdbeere, Wasserphase |
| | 2.000 g | Osmoselösung 60 ° Brix Kaliumlactat |

Konzentrierung der Aromastoffe um den Faktor 20

| Apparatur: | Forward-Osmose-Modul von Aquaporin LHF033 kombiniert mit 10 Liter-Tank von Sartorius + 2 Ismatec Pumpen |
|---|---|
| Membran: | FO Modul Aquaporin LHF033, 0,6 m$^2$ aktive Membranfläche |
| Versuchsdurchführung: | Der Feed-Tank wurde mit 10.000g Wasserphase befüllt und 2,0 kg Draw Solution (60° Brix) im Kanister vorgegeben. Danach wurde die Feed-Pumpe und die Draw |

Solution Pumpe mit jeweils einem Fluss von 300 ml/min gestartet.

Auswaagen:

Ausgang: 10.000 g
Retentat: 510,0 g
Permeat: 9.500 g

Draw Solution Start: 60 ° Brix

Draw Solution Ende: 12,4 ° Brix

Temperatur: 25°C

Aufzeichnung der Daten:

| Zeit (min) | | 0 | 15 | 30 | 60 | 90 |
|---|---|---|---|---|---|---|
| Menge des zugesetzten Produktes (g) | 10.000 | | | | | |
| Menge der zugesetzten Draw Solution (kg) | 2,0 | | | | | |
| Gewicht Draw Solution (kg) | | 0 | 1.890 | 3.638 | 6.054 | 7.990 |
| Flux (l/m$^2$·h) | | | 12,6 | 11,7 | 8,1 | 6,5 |

| Zeit (min) | 120 | 123 Ende | | | |
|---|---|---|---|---|---|
| Menge des zugesetzten Produktes (g) | | | | | |
| Menge der zugesetzten Draw Solution (kg) | | | | | |
| Gewicht Draw Solution (kg) | 9.440 | 9.500 | | | |
| Flux (l/m$^2$·h) | 4,8 | 4,0 | | | |

Das sensorische Profil ist wie folgt:

Intensität: 1 - 10

| | Ausgang Erdbeere, Wasserphase | Konzentrat nach Rückverdünnung |
|---|---|---|
| Fruchtig-estrig | 6 | 5 |
| Buttrig | 4 | 5 |
| Grün | 5 | 5 |
| Reif | 4 | 4 |
| Saftig | 6 | 6 |
| Gekocht | 0 | 0 |

Das sensorische Profil ist in Figur 6 wiedergegeben.

**Beispiel 4 (nicht erfindungsgemäß): Herstellung eines Konzentrates von schwarzem Tee, Ceylon**

Produkt:   Schwarzer Tee aus Ceylon
Einsatz:   600 g   Tee

(fortgesetzt)

| 12.000 g | Wasser |
|---|---|
| 2.000 g | Osmoselösung 60 ° Brix Kaliumlactat |

Konzentrierung der Aromastoffe um den Faktor 20

| | |
|---|---|
| Apparatur: | Forward-Osmose-Modul von Aquaporin LHF033 kombiniert mit 10 Liter-Tank von Sartorius + 2 Ismatec Pumpen |
| Membran: | FO Modul Aquaporin LHF033, 0,6m$^2$ aktive Membranfläche |
| Versuchsvorbereitung: | 600 g Tee wurden für 2 Stunden bei Raumtemperatur in 12 kg Wasser extrahiert, danach über Sieb und Faltenfilter von Schwebstoffen befreit. 10 kg der Wasserphase wurden für die Forward-Osmose eingesetzt. |
| Versuchsdurchführung: | Der Feed-Tank wurde mit 10.000g Wasserphase befüllt und 2,0 kg Draw Solution (60 ° Brix) im Kanister vorgegeben. Danach wurde die Feed-Pumpe und die Draw Solution Pumpe mit jeweils einem Fluss von 300 ml/min gestartet. |

Auswaagen:

| | |
|---|---|
| Ausgang: | 10.000 g, 1,3 ° Brix |
| Retentat: | 278,1 g, 33,1 ° Brix |
| Permeat: | 9.602 g |

Draw Solution I Start: 60 ° Brix
Draw Solution II Start: 60 ° Brix
Draw Solution Ende: 18,4 ° Brix
Draw Solution II Ende: 21,9 ° Brix

Aufzeichnung der Daten:

| Zeit (min) | 0 | 15 | 30 | 60 | 120 |
|---|---|---|---|---|---|
| Menge des zugesetzten Produktes (g) | 10.000 | | | | |
| Menge der zugesetzten Draw Solution (kg) | 2,0 | | | | |
| Gewicht Draw Solution (kg) | 0 | 2.028 | 2.880 | 3.760 | 4.744 |
| Flux (l/m$^2$·h) | | 13,5 | 5,7 | 2,9 | 1,6 |

| Zeit (min) | 180 | 240 | 300 | 344 | |
|---|---|---|---|---|---|
| Menge des zugesetzten Produktes (g) | | | | | |
| Menge der zugesetzten | | 2,0 | | | |

(fortgesetzt)

| Zeit (min) | 180 | 240 | 300 | 344 | |
|---|---|---|---|---|---|
| Draw Solution (kg) | | | | | |
| Gewicht Draw Solution (kg) | 5.569 | 2.068 | 3.510 | 4.033 | |
| Flux (l/m2·h) | 1,3 | 7,6 | 2,4 | 1,2 | |

**Patentansprüche**

1. Verfahren zur Herstellung eines Aromastoff-Konzentrates, umfassend die folgenden Schritte:

   - Bereitstellen einer wässrigen Ausgangslösung aus einem Lebensmittel, wobei die wässrige Ausgangslösung ein Brüdenkondensat aus Obst oder Gemüse ist,
   - Aufkonzentration derwässrigen Ausgangslösung durch Osmose mit einer semipermeablen biomimetischen Membran, und
   - Bildung eines Aromastoff-Konzentrats als Retentat,

   worin die semipermeable biomimetische Membran umfasst:

   Vesikel aus Liposomen oder Polymersomen, in die jeweils mindestens ein Aquaporin-Protein eingebaut ist;
   eine Dünnfilm-Verbundmatrix, in der die Vesikel eingebettet sind; und
   eine Trägerschicht;
   wobei das Verfahren bei einer Temperatur im Bereich von 10 bis 40 °C durchgeführt wird; und
   worin die wässrige Ausgangslösung um den Faktor 20 bis 1.000 aufkonzentriert wird.

2. Verfahren nach Anspruch 1, worin das Liposom umfasst: Lipide wie DPhPC, DOPC, gemischte Sojabohnen-Lipide, Asolectin oder gemischte Lipide von E. coli; und worin das Polymersom umfasst: Triblockcopolymere des hydrophilen-hydrophoben-hydrophilen Typs A-B-A, A-B-C oder CBA, worin A steht für PMOXA, B steht für PDMS und C steht für PEO, Diblockcopolymere des hydrophilen-hydrophoben Typs A-B, worin A steht für PB und B steht für PEO, oder Kombinationen daraus.

3. Verfahren nach einem der Ansprüche 1 oder 2, worin das Aquaporin-Protein ausgewählt ist aus der Gruppe, die besteht aus AQPO, AqpZ, SoPIP2 AQP10 und deren Isoformen, vorzugsweise worin das Aquaporin-Protein AQPO ist.

4. Verfahren nach einem der Ansprüche 1 bis 3, worin die Dünnfilm-Verbundmatrix hergestellt wird durch Polymerisation einer wässrigen Lösung eines Amins mit einer Lösung eines Säurechlorids in einem organischen Lösungsmittel.

5. Verfahren nach einem der vorangehenden Ansprüche 1 bis 4, worin die wässrige Ausgangslösung mindestens um den Faktor 100 aufkonzentriert wird.

6. Aromastoff-Konzentrat, erhältlich nach einem Verfahren der vorangehenden Ansprüche 1 bis 5, das kein Off-flavour aufweist und frei ist von störenden Aromakomponenten, insbesondere frei ist von störenden Aromakomponenten mit einem OAV (odor activity value) $\geq$ 1.

7. Aromastoff-Konzentrat nach Anspruch 6, worin das Aromaprofil eines rückverdünnten Konzentrats aus der Osmose mit der semipermeablen biomimetischen Membran vom Aromaprofil einer wässrigen Ausgangslösung bei einer Skala von 0 bis 10 Punkten in den 6 Geschmacksachsen eines konventionellen Panel-Profils um insgesamt max. 1 Punkt abweicht, wobei das Aromaprofil über ein Testpanel gemessen wird nach DIN 10967-1-1999 oder erfindungsgemäßem Protokoll.

8. Aromastoff-Konzentrat nach einem der Ansprüche 6 bis 7, bei dem der Ethanol-Gehalt um mindestens 50 % reduziert ist, bezogen auf die wässrige Ausgangslösung.

9. Aromastoff-Konzentrat nach Anspruch 6, das je nach Ausgangslösung frei ist von Störkomponenten, ausgewählt aus den Gruppen, die enthalten:

| | |
|---|---|
| Bei Ananas: | Methional, 2,4-(E,E)-Decadienal |
| Bei Apfel: | 2,3-Methylbutyraldehyd, Furfural, 1,3-Pentanal, 3,5-Octadienal, Linalooloxid, 8-p-Cymenol |
| Bei Citrusfrüchten: | $\alpha$-Terpineol, p-Dimethylstyrol, Limonenepoxid, Carveol, Carvon, 4-Vinylguaiacol |
| Bei Erdbeere: | $\beta$-Damascenon, 2,4-(E,E)-Decadienal, Furfural, Guajacol |
| Bei Passionsfrucht: | Benzaldehyd, $\alpha$-Terpineol, Furfural, Essigsäure, Acetophenon, $\beta$-Ionon, Linalooloxid |
| Bei Tomate: | Dimethyldisulfid, Methional, 4-Hydroxy-2,5-dimethyl-3(2H)-furanon |
| Bei Zwiebelsaft: | Dimethyltrisulfid, Dipropyltrisulfid, Dimethyltetrasulfid, 3,5-Diethyl-1,2,4-trithiolan, Thiophen |

10. Aromastoff-Konzentrat nach einem der vorangehenden Ansprüche 6 bis 9, das mindestens 100-fold ist, insbesondere 200- bis 1.000-fold ist.

11. Verwendung des Aromastoff-Konzentrats nach einem der vorangehenden Ansprüche 6 bis 10 zur Aromatisierung oder zur Rekonstitution des Aromas von Lebensmitteln, Getränkeprodukten, Halbfertigprodukten, Mundhygieneprodukten, kosmetischen oder pharmazeutischen Produkten.

## Claims

1. Method for manufacturing of a flavouring concentrate comprising the following steps:

   - preparing an aqueous starting solution from foodstuff, wherein the aqueous starting solution being a vapour condensate from fruits or vegetables,
   - concentrating the aqueous starting solution by osmosis using a semipermeable biomimetic membrane, and
   - forming a flavouring concentrate as retentate,

   wherein the semipermeable biomimetic membrane comprises:

   vesicles of liposomes or polymersomes containing at least one aquaporine protein;
   a thin-film composite matrix containing embedded vesicles; and
   a carrier layer;
   wherein the method is performed at a temperature in a range of 10 to 40°C; and
   wherein the aqueous starting solution is concentrated by the factor 20 to 1000.

2. Method according to claim 1, wherein the liposom comprises: lipids like DPhPC, DOPC, mixed soybean lipids, asolectin or mixed lipids of *E. coli;* and wherein the polymersome comprises: triblock copolymers of the hydrophilic-hydrophobic-hydrophilic type A-B-A, A-B-C or CBA, wherein A stands for PMOXA, B stands for PDMS and C stands for PEO, diblock copolymers of the hydrophilic-hydrophobic type A-B, wherein A stands for PB and B stands for PEO, or combinations thereof.

3. Method according to any one of claims 1 or 2, wherein the aquaporine protein is selected from the group consisting of AQPO, AqpZ, SoPIP2 AQP10 and isoforms thereof, preferably wherein the aquaporine protein is AQPO.

4. Method according to any one of claims 1 to 3, wherein the thin-film composite matrix is prepared by polymerisation of an aqueous solution of an amine with a solution of an acid chloride in an organic solvent.

5. Method according to any one of claims 1 to 4, wherein the aqueous starting solution is concentrated by at least the factor 100.

6. Flavouring concentrate obtainable by a method according to any one of the previous claims 1 to 5 exhibiting no off-flavour and being free of disturbing flavouring components, especially being free of disturbing aroma components with an OAV (odor activity value) $\geq 1$.

7. Flavouring concentrate according to claim 6, wherein the aromatic profile of a diluted concentrate from the osmosis with a semipermeable biomimetic membrane differs by a maximum of 1 point from the aromatic profile of an aqueous starting solution, using a scale of 0 to 10 points to evaluate the 6 flavour axes of a conventional panel profile, wherein the aromatic profile is measured by a testing panel according to DIN 10967-1-1999 or a protocol in accordance to the invention.

8. Flavouring concentrate according to any one of claims 6 to 7, wherein the ethanol content is reduced by at least 50% in relation to the aqueous starting solution.

9. Flavouring concentrate according to claim 6, which is free of disturbing substances depending on the starting solutions, selected from the groups containing:

> For pineapple: methional, 2,4-(E,E)-decadienal
> For apple: 2,3-methyl butyraldehyde, furfural, 1,3-pentanal, 3,5-octadienal, linalool oxide, 8-p-cymenol
> For citrus fruits: $\alpha$-terpineol, p-dimethyl styrene, limonene epoxide, carveol, carvone, 4-vinyl guaiacol
> For strawberry: $\beta$-damascenone, 2,4-(E,E)-decadienal, furfural, guaiacol
> For passion fruit: benzaldehyde, $\alpha$-terpineol, furfural, acetic acid, acetophenone, $\beta$-ionone, linalool oxide
> For tomato: dimethyl disulfide, methional, 4-hydroxy-2,5-dimethyl-3(2H)-furanone
> For onion juice: dimethyl trisulfide, dipropyl trisulfide, dimethyl tetrasulfide, 3,5-diethyl-1,2,4-trithiolane, thiophene

10. Flavouring concentrate according to any one of the previous Claims 6 to 9, which is at least 100-fold, preferably 200-fold to 1000-fold.

11. Use of the flavouring concentrate according to any one of the previous claims 6 to 10 for aromatisation or reconstitution of the aroma in foodstuff, beverages, semifinished products, oral hygiene products, cosmetic or pharmaceutical products.


**Revendications**

1. Procédé de production d'un concentré de substance aromatisante comprenant les étapes suivantes consistant à:

> - fournir une solution aqueuse de départ à partir d'un aliment, dans lequel la solution aqueuse de départ est un condensat de buées de fruit ou de légume,
> - concentrer la solution aqueuse de départ par osmose avec une membrane biomimétique semi-perméable, et
> - former un concentré de substance aromatisante en tant que rétentat,

> dans lequel la membrane biomimétique semi-perméable comprend:

> des vésicules constituées de liposomes ou de polymersomes dans lesquels est intégrée au moins une protéine aquaporine,
> une matrice composite à film mince dans laquelle les vésicules sont noyées, et
> une couche de support,
> dans lequel le procédé est mis en oeuvre à une température comprise entre 10 et 40 °C; et
> dans lequel la solution aqueuse de départ est concentrée du facteur de 20 à 1.000.

2. Procédé selon la revendication 1, dans lequel le liposome comprend: des lipides tels que le DPhPC, le DOPC, les lipides mixtes de soja, de l'asolectine ou des lipides mixtes d'E. coli, et dans lequel le polymersome comprend: des copolymères triblocs du type hydrophile-hydrophobe-hydrophile A-B-A, A-B-C ou CBA, dans lesquels A signifie PMOXA, B signifie PDMS et C signifie PEO, des copolymères diblocs du type hydrophile-hydrophobe A-B, dans lesquels A signifie PB et B signifie PEO, ou des combinaisons de ceux-ci.

3. Procédé selon l'une quelconque des revendications 1 ou 2, dans lequel la protéine aquaporine est choisie dans le groupe qui est constitué de AQPO, AqpZ, SoPIP2 AQP10 et de leurs isoformes, de préférence dans lequel la protéine aquaporine est AQPO.

4. Procédé selon l'une quelconque des revendications 1 à 3, dans lequel la matrice composite à film mince est préparée par polymérisation d'une solution aqueuse d'une amine avec une solution d'un chlorure d'acide dans un solvant organique.

5. Procédé selon l'une quelconque des revendications précédentes 1 à 4, dans lequel la solution aqueuse de départ est concentrée au moins du facteur 100.

6. Concentré de substance aromatisante, apte à être obtenu par un procédé selon les revendications 1 à 5 précédentes, qui ne présente aucun défaut d'arôme et est exempt de composants aromatiques perturbateurs, en particulier est exempt de composants aromatiques perturbateurs avec une OAV (valeur d'activité odorante) $\geq$ 1.

7. Concentré de substance aromatisante selon la revendication 6, dans lequel le profil aromatique d'un concentré redilué issu de l'osmose avec la membrane biomimétique semi-perméable s'écarte d'un total de 1 point au maximum du profil aromatique d'une solution aqueuse de départ sur une échelle de 0 à 10 points dans les 6 axes de goût d'un profil de panneau conventionnel, dans lequel le profil aromatique est mesuré via un panneau de test selon la norme DIN 10967-1-1999 ou le protocole selon l'invention.

8. Concentré de substance aromatisante selon l'une quelconque des revendications 6 à 7, dans lequel la teneur en éthanol est réduite d'au moins 50 %, par rapport à la solution aqueuse de départ.

9. Concentré de substance aromatisante selon la revendication 6, qui, selon la solution de départ, est exempt de composants perturbateurs, choisis dans les groupes qui contiennent:

| | |
|---|---|
| dans l'ananas: | le méthional, le 2,4-(E,E)-décadiénal |
| dans la pomme: | le 2,3-méthylbutyraldéhyde, le furfural, le 1,3-pentanal, le 3,5-octadiénal, l'oxyde de linalol, le 8-p-cyménol |
| dans les agrumes: | l'$\alpha$-$\alpha$-terpinéol, le p-diméthylstyrène, l'époxyde de limonène, le carvéol, la carvone, le 4-vinylguaiacol |
| dans la fraise: | la $\beta$-damascénone, le 2,4-(E,E)-décadienal, le furfural, le gaïacol |
| dans le fruit de la passion: | le benzaldéhyde, l'a-terpinéol, le furfural, l'acide acétique, l'acétophénone, la $\beta$-ionone, l'oxyde de linalol |
| dans la tomate: | le disulfure de diméthyle, le méthional, la 4-hydroxy-2,5-diméthyl-3(2H)-furanone |
| dans le jus d'oignon: | le trisulfure de diméthyle, le trisulfure de dipropyle, le tétrasulfure de diméthyle, le 3,5-diéthyl-1,2,4-trithiolane, le thiophène |

10. Concentré de substance aromatisante selon l'une quelconque des revendications 6 à 9 précédentes, qui est au moins 100 fois, en particulier 200 à 1.000 fois.

11. Utilisation du concentré de substance aromatisante selon l'une quelconque des revendications 6 à 10 précédentes, pour aromatiser ou pour reconstituer l'arôme d'aliments, de produits de boisson, de produits semi-finis, de produits d'hygiène bucco-dentaire, de produits cosmétiques ou pharmaceutiques.

**Figur 1: Aufbau Aquaporin-Membran**

**Figur 2: Prinzip Forward-Osmose**

**Figur 3: Forward-Osmose mit Aquaporin-Membran am Beispiel eines Kaffee-Extraktes**

**Figuren 4a und 4b:** Chromatogrammvergleich von einem Teeextrakt oben und dem zurückverdünnten Konzentrat des gleichen Teeextrakts unten

Figur 5: Gelber Pfirsich: Sensorische Profilierung von Wasserphase und rückverdünntem Konzentrat aus der Aquaporin-Membranfiltration

Figur 6: Erdbeere: Sensorische Profilierung von Wasserphase und rückverdünntem Konzentrat aus der Aquaporin-Membranfiltration

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2075320 A1
- EP 0082284 A1
- WO 2017080852 A1
- WO 2014108827 A1
- US 2016016127 A1
- US 2017259217 A1
- US 2012152841 A1
- US 4952751 A
- DK 201770753 A1

**In der Beschreibung aufgeführte Nicht-Patentliteratur**

- **BRADDOCK, R.J.** Ultrafiltration and Reverse Osmosis Recovery of Limonene from Citrus Processing Waste Streams. *Journal of Food Science,* 1982, vol. 47 (3), 946-948
- **TANG et al.** Desalination by biomimetic aquaporin membranes: Review of status and prospects. *Desalination,* 2013, vol. 308
- **JOACHIM HABEL et al.** *Membranes "Aquaporin-based biomimetic polymeric mambrances: approaches and challenges,* 2015, vol. 5, 307-351